# EUROPEAN PATENT SPECIFICATION

(11) **EP 1 723 933 B1**
(45) Date of publication and mention of the grant of the patent: **19.11.2008**
(21) Application number: 06015132.1
(22) Date of filing: 25.01.2002
(51) Int. Cl.: A61F 2/16

(54) **Accommodating intraocular lens system**
Akkommodierendes Intraokularlinsensystem
Système de lentille intraoculaire capable de s'accommoder

(30) Priority: 25.01.2001 US 264179 P; 13.04.2001 US 283856 P; 09.11.2001 US 337343 P; 11.12.2001 US 20853; 11.12.2001 US 20858; 11.12.2001 US 17916; 11.12.2001 US 17920; 11.12.2001 US 21797; 11.12.2001 US 17915; 11.12.2001 US 21795; 11.12.2001 US 17753; 11.12.2001 US 20002
(43) Date of publication of application: 22.11.2006
(62) Divisional of application: 02718880.4
(73) Proprietor: Zadno-Azizi, Gholam-Reza, Fremont, CA 94539 (US); Rogers, Erica J., Saratoga, CA 95070 (US); Nguyen, Tuan Anh, Orange, CA 92869 (US); Ting, Albert C., Laguna Niguel, CA 92617-1382 (US); Portney, Valdemar, Tustin, CA 92782 (US); Pham, Hai-Minh, Huntington Beach, CA 92646 (US)
(72) Inventor: Zadno-Azizi, Gholam-Reza, Fremont, CA 94539 (US); Rogers, Erica J., Saratoga, CA 95070 (US); Nguyen, Tuan Anh, Orange, CA 92869 (US); Ting, Albert C., Laguna Niguel, CA 92617-1382 (US); Portney, Valdemar, Tustin, CA 92782 (US); Pham, Hai-Minh, Huntington Beach, CA 92646 (US)
(74) Representative: Illingworth-Law, William Illingworth

(56) References cited:
- EP-A- 0 337 390
- WO-A-00/66037
- US-A- 4 892 543
- US-A- 5 443 506

## Description

### Background of the Invention

### Field of the invention

This invention relates to intraocular lenses and, more particularly, to intraocular lenses that alter the refractive power of the eye in response to changes in the tension of the ciliary muscle of the eye.

### Description of the Related Art

The vast majority of cataract operations involve the Implantation of an artificial lens following cataract removal. Typically these lenses have a fixed focal length or, in the case of bifocal or multifocal lenses, have several different fixed focal lengths. Such fixed focal-length lenses lack the ability of the natural lens to dynamically change the refractive power of the eye. The various embodiments of the intraocular lens disclosed herein provide an accommodating lens system which alters the refractive power of the eye in response to changes in tension of the ciliary muscle, thereby allowing the lens system to bring into focus on the retina images of objects that are both near and far from the eye.
WO 00/66037 (Allergan Sales, Inc) relates to an intraocular lens combination which includes a first optic having a negative optical power and being adapted to be placed in a substantially fixed position in a mammalian eye. A second optic having a higher optical power than the first optic is also provided in addition, a movement assembly is provided which is coupled to the second optic and is adapted to cooperate with the eye to effect accommodating movement of the second optic in the eye. The intraocular lens combination can be effectively positioned to effectively inhibit or reduce the risk of posterior capsular opacification.
US 4,892,543 (Turley et al) relates to intraocular lens providing accommodation.
EP 0337390 (Ceskoslovenska Akademie Ved) relates to an intraocular optical system.
US 5,443,506 (Garabet et al) relates to a lens with variable optical properties.

### Summary of the Invention

One aspect of the invention is an accommodating intraocular lens for implantation in an eye having an optical axis. The lens comprises an anterior portion which in turn comprises an anterior viewing element comprised of an optic having refractive power and an anterior biasing element comprising first and second anterior translation members extending from the anterior viewing element. The lens further comprises a posterior portion which in turn comprises a posterior viewing element in spaced relationship to the anterior viewing element and a posterior biasing element comprising first and second posterior translation members extending from the posterior viewing element. The anterior portion and posterior portion meet at first and second apices of the intraocular lens such that a plane perpendicular to the optical axis and passing through the apices is closer to one of said viewing elements than to the other of said viewing elements. The anterior portion and the posterior portion are responsive to force thereon to cause the separation between the viewing elements to change.

The present invention discloses an accomodating lens as set forth in claim 1.

### Brief Description of the Drawings

Having thus summarized the general nature of the invention, certain preferred embodiments and modifications thereof will become apparent to those skilled in the art from the detailed description herein having reference to the figures that follow, of which:
Figure 1 is a sectional view of the human eye, with the lens in the unaccommodated state.
Figure 2 is a sectional view of the human eye, with the lens in the accommodated state.
Figure 3 is a perspective view of one embodiment of an intraocular lens system.
Figure 4 is a side view of the lens system.
Figure 5 is a rear perspective view of the lens system.
Figure 6 is a front view of the lens system.
Figure 7 is a rear view of the lens system.
Figure 8 is a top view of the lens system.
Figure 9 is a side sectional view of the lens system.
Figure 10 is a top sectional view of the lens system.
Figure 11 is a second perspective view of the lens system.
Figure 12 is a third perspective view of the lens system.
Figure 13 is a side view of the lens system in the unaccommodated state.
Figure 14 is a side sectional view of the lens system in the unaccommodated state.
Figure 15 is a top sectional view of the lens system in the unaccommodated state.
Figure 16 is a sectional view of the human eye with the lens system implanted in the capsular bag and the lens system in the accommodated state.
Figure 17 is a sectional view of the human eye with the lens system implanted in the capsular bag and the lens system in the unaccommodated state.
Figure 17.1 is a sectional view of an arm of the lens system.
Figure 17.2 is a sectional view of another embodiment of the arm of the lens system.
Figure 17.3 a sectional view of other embodiments of the arm of the lens system.
Figure 17.4 is a side sectional view of another embodiment of the lens system.
Figure 17.5 is a side sectional view of another embodiment of the lens system.
Figure 18 is a side view of another embodiment of the lens system.
Figure 19 is a side sectional view of another embodiment of the lens system.
Figure 20 is a rear perspective view of another embodiment of the lens system.
Figure 21 is a partial top sectional view of another embodiment of the lens system, implanted in the capsular bag.
Figure 21.1 is a front view of another embodiment of the lens system.
Figure 21.2 is a front view of another embodiment of the lens system.
Figure 21.3 is a front view of another embodiment of the lens system.
Figure 22 is a partial side sectional view of another embodiment of the lens system, implanted in the capsular bag.
Figure 22.1 is a side view of a stop members system employed in one embodiment of the lens system
. Figure 23 is a side view of a mold system for forming the lens system.
Figure 24 is a side sectional view of the mold system.
Figure 25 is a perspective view of a first mold portion.
Figure 26 is a perspective view of a second mold portion.
Figure 27 is a top view of the second mold portion.
Figure 28 is a side sectional view of the second mold portion.
Figure 29 is another side sectional view of the second mold portion.
Figure 30 is a bottom view of a center mold portion.
Figure 31 is a top view of the center mold portion.
Figure 32 is a sectional view of the center mold portion.
Figure 33 is another sectional view of the center mold portion.
Figure 34 is a perspective view of the center mold portion.
Figure 34.1 is a partial cross sectional view of an apex of the lens system, showing a set of expansion grooves formed therein.
Figure 35 is a schematic view of another embodiment of the lens system.
Figure 36 is a schematic view of another embodiment of the lens system.
Figure 37 is a perspective view of another embodiment of the lens system.
Figure 38 is a top view of another embodiment of the lens system.
Figure 38.1 is a schematic view of another embodiment of the lens system, as implanted in the capsular bag.
Figure 38.2 is a schematic view of the embodiment of Figure 38.1, in the accommodated state.
Figure 38.3 is a schematic view of biasers installed in the lens system.
Figure 38.4 is a schematic view of another type of biasers installed in the lens system.
Figure 38.5 is a perspective view of another embodiment of the lens system.
Figure 39 is a series of schematic views of an insertion technique for use in connection with the lens system
Figure 40 is a schematic view of fluid-flow openings formed in the anterior aspect of the capsular bag.
Figure 40.1 is a front view of the lens system, illustrating one stage of a folding technique for use with the lens system.
Figure 40.2 is a front view of the lens system, illustrating another stage of the folding technique.
Figure 40.3 illustrates another stage of the folding technique.
Figure 40.4 illustrates another stage of the folding technique.
Figure 40.5 illustrates another stage of the folding technique.Figure 40.6 illustrates another stage of the folding technique.
Figure 40.7 is a perspective view of a folding tool for use with the lens system.
Figure 41 is a sectional view of an aspheric optic for use with the lens system.
Figure 42 is a sectional view of an optic having a diffractive surface for use with the lens system.
Figure 43 is a sectional view of a low-index optic for use with the lens system.

### Detailed Description of the Preferred Embodiment

### I. THE HUMAN EYE AND ACCOMMODATION

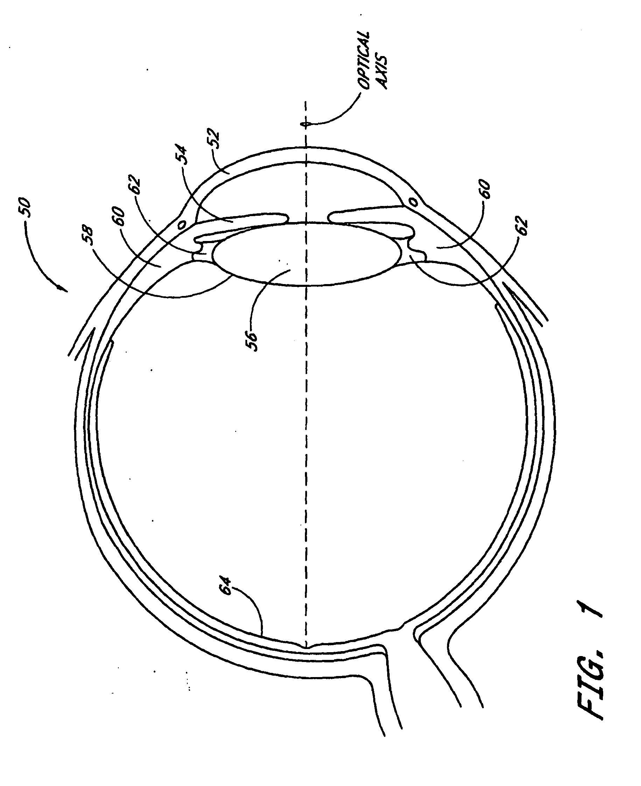

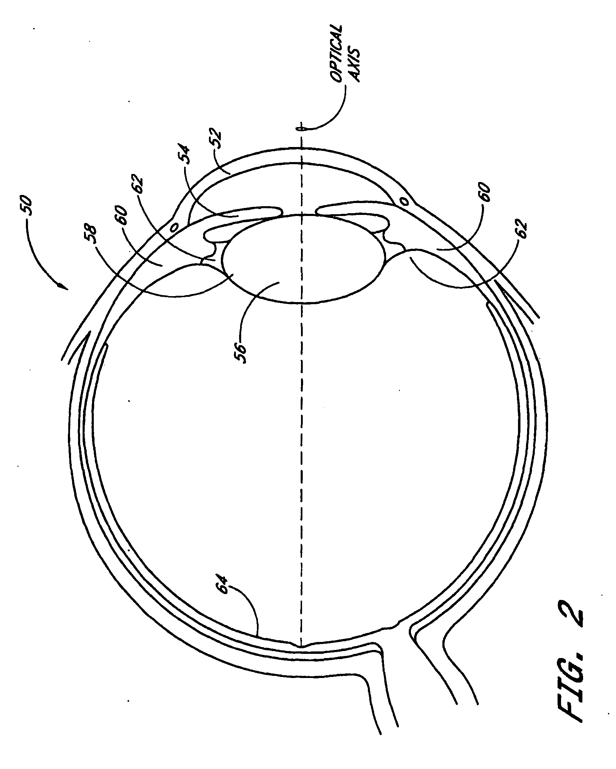

Figures 1 and 2 show the human eye 50 in section. Of particular relevance to the present disclosure are the cornea 52, the iris 54 and the lens 56, which is situated within the elastic, membranous capsular bag or lens capsule 58. The capsular bag 58 is surrounded by and suspended within the ciliary muscle 60 by ligament-like structures called zonules 62.

As light enters the eye 50, the cornea 52 and the lens 56 cooperate to focus the incoming light and form an image on the retina 64 at the rear of the eye, thus facilitating vision. In the process known as accommodation, the shape of the lens 56 is altered (and its refractive properties thereby adjusted) to allow the eye 50 to focus on objects at varying distances. A typical healthy eye has sufficient accommodation to enable focused vision of objects ranging in distance from infinity (generally defined as over 20 feet from the eye) to very near (closer than 10 inches).

The lens 58 has a natural elasticity, and in its relaxed state assumes a shape that in cross-section resembles a football. Accommodation occurs when the ciliary muscle 60 moves the lens from its relaxed or "unaccommodated" state (shown in Figure 1) to a contracted or "accommodated" state (shown in Figure 2). Movement of the ciliary muscle 60 to the relaxed/unaccommodated state increases tension in the zonules 62 and capsular bag 58, which in turn causes the lens 56 to take on a thinner (as measured along the optical axis) or taller shape as shown in Figure 1. In contrast, when the ciliary muscle 60 is in the contracted/accommodated state, tension in the zonules 62 and capsular bag 58 is decreased and the lens 56 takes on the fatter or shorter shape shown in Figure 2. When the ciliary muscles 60 contract and the capsular bag 58 and zonules 62 slacken, some degree of tension is maintained in the capsular bag 58 and zonules 62.

### II. THE LENS SYSTEM: STRUCTURE

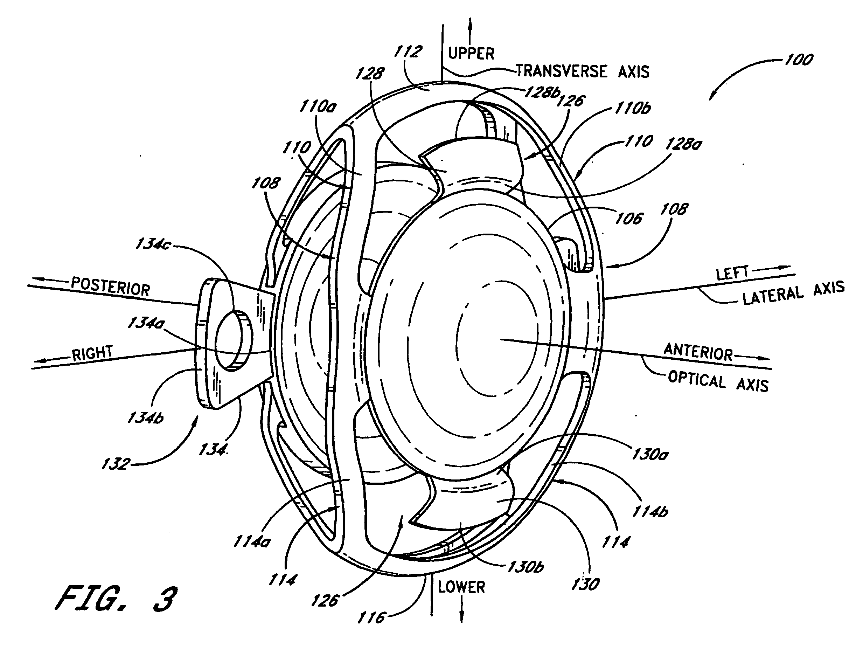

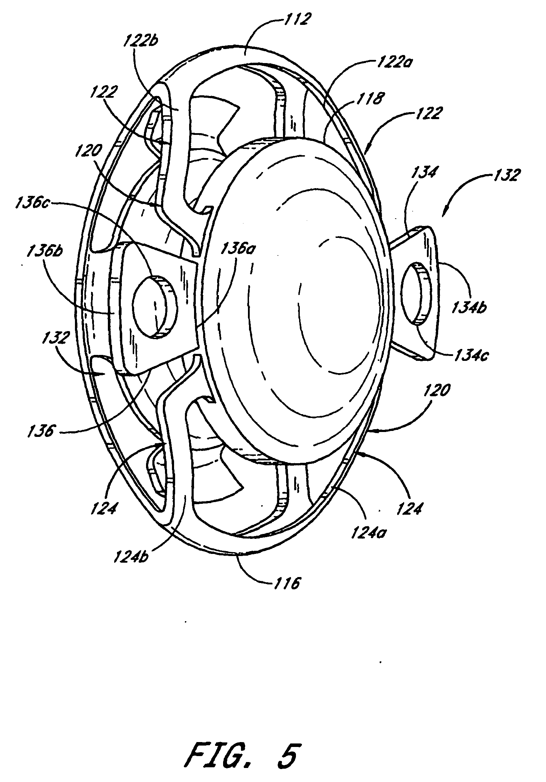

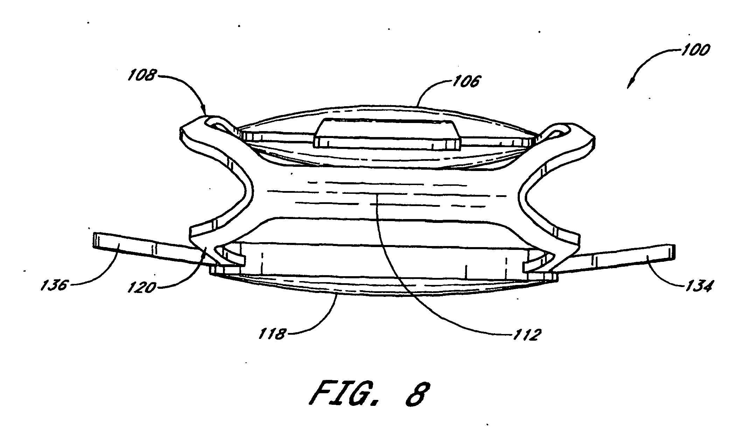

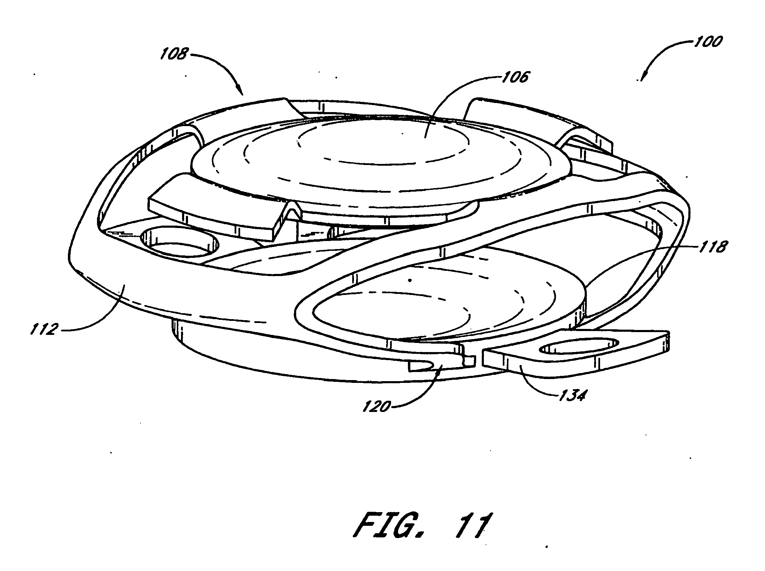

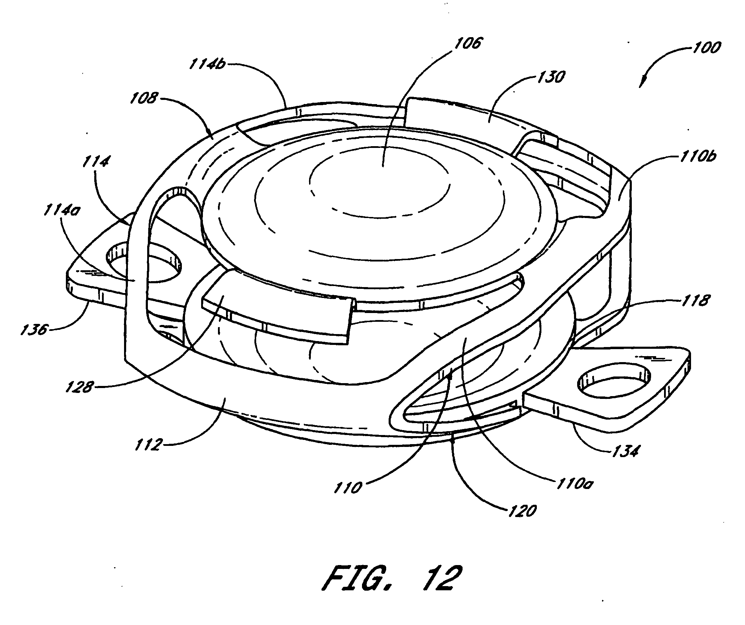

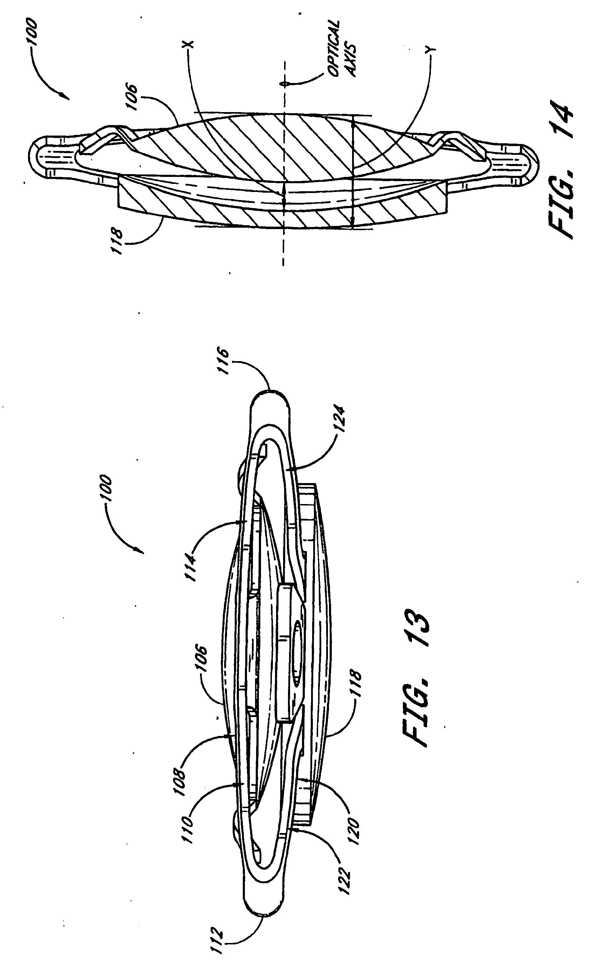

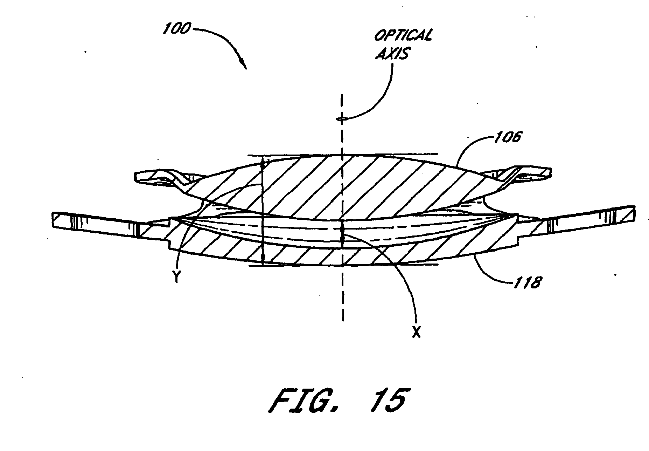

Figures 3-17 depict one embodiment of an intraocular lens system 100 which is configured for implantation into the capsular bag 58 in place of the natural lens 56, and is further configured to change the refractive properties of the eye in response to the eye's natural process of accommodation. With reference to Figure 3, a set of axes is included to illustrate the sense of directional terminology which will be used herein to describe various features of the lens system 100. The terms "anterior" and "posterior" refer to the depicted directions on the optical axis of the lens 100 shown in Figure 3. When the lens 100 is implanted in an eye, the anterior direction extends toward the cornea and the posterior direction extends toward the retina, with the optical axis of the lens substantially coincident with the optical axis of the eye shown in Figures 1 and 2. The terms "left" and "right" refer to the directions shown on the lateral axis, which is orthogonal to the optical axis. In addition, the terms "upper" and "lower" refer to the directions depicted on the transverse axis which is orthogonal to both of the optical axis and the lateral axis.

This system of axes is depicted purely to facilitate description herein; thus, it is not intended to limit the possible orientations which the lens system 100 may assume during use. For example, the lens system 100 may rotate about, or may be displaced along, the optical axis during use without detracting from the performance of the lens. It is clear that, should the lens system 100 be so rotated about the optical axis, the transverse axis may no longer have an upper-lower orientation and the lateral axis may no longer have a left-right orientation, but the lens system 100 will continue to function as it would when oriented as depicted in Figure 3. Accordingly, when the terms "upper," "lower," "left" or "right" are used in describing features of the lens system 100, such use should not be understood to require the described feature to occupy the indicated position at any or all times during use of the lens system 100. Similarly, such use should not be understood to require the lens system 100 to maintain the indicated orientation at any or all times during use.

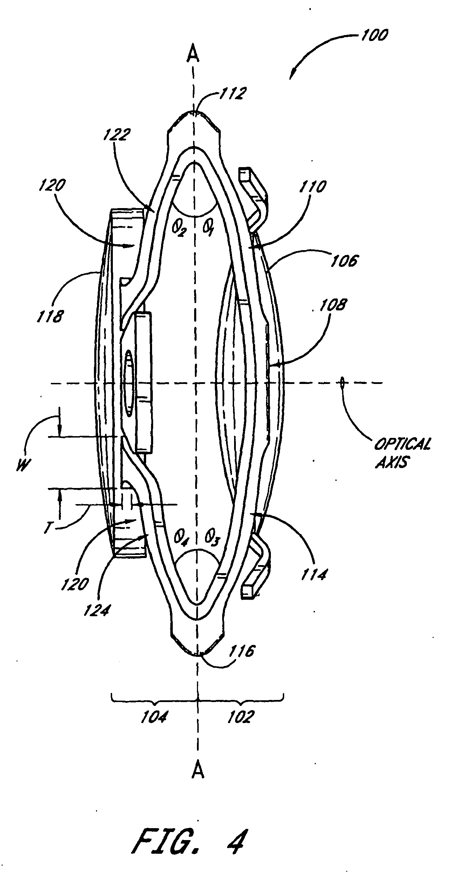

As best seen in Figure 4, the lens system 100 has an anterior portion 102 which is anterior or forward of the line A-A (which represents a plane substantially orthogonal to the optical axis and intersecting first and second apices 112, 116) and a posterior portion 104 which is posterior or rearward of the line A-A. The anterior portion 102 comprises an anterior viewing element 106 and an anterior biasing element 108. The anterior biasing element 108 in turn comprises a first anterior translation member 110 which extends from the anterior viewing element 106 to the first apex 112 and a second anterior translation member 114 which extends from the anterior viewing element 106 to the second apex 116. In the illustrated embodiment the first anterior translation member 110 comprises a right arm 110a and a left arm 110b (see Figure 3). In addition, the depicted second anterior translation member 114 comprises a right arm 114a and a left arm 114b. However, in other embodiments either or both of the first and second anterior translation members 110, 114 may comprise a single arm or member, or more than two arms or members.

As best seen in Figures 4, 5 and 7, the posterior portion 104 includes a posterior viewing element 118 and a posterior biasing element 120. The posterior biasing element 120 includes a first posterior translation member 122 extending from the posterior viewing element 118 to the first apex 112 and a second posterior translation member 124 extending from the posterior viewing element 118 to the second apex 116. In the illustrated embodiment, the first posterior translation member comprises a right arm 122a and a left arm 122b. Likewise, the depicted second posterior translation member 124 comprises a right arm 124a and a left arm 124b. However, in other embodiments either or both of the first and second posterior translation members 122, 124 may comprise a single arm or member, or more than two arms or members.

In the embodiment shown in Figure 4, the anterior biasing element 108 and the posterior biasing element are configured symmetrically with respect to the plane A-A as the lens system 100 is viewed from the side. As used herein to describe the biasing elements 108, 120, "symmetric" or "symmetrically" means that, as the lens system 100 is viewed from the side, the first anterior translation member 110 and the first posterior translation member 122 extend from the first apex 112 at substantially equal first anterior and posterior biasing angles 1, 2 with respect to the line A-A (which, again, represents the edge of a plane which is substantially orthogonal to the optical axis and intersects the first and second apices 112, 116) and/or that the second anterior translation member 114 and the second posterior translation member 124 extend from the second apex 116 at substantially equal second anterior and posterior biasing angles 3, 4 with respect to the line A-A. Alternative or asymmetric configurations of the biasing elements are possible, as will be discussed in further detail below. It should be further noted that a symmetric configuration of the biasing elements 108, 120 does not dictate symmetric positioning of the viewing elements with respect to the line A-A, in the embodiment shown in Figure 4 the anterior viewing element 106 is closer to the line A-A than is the posterior viewing element.

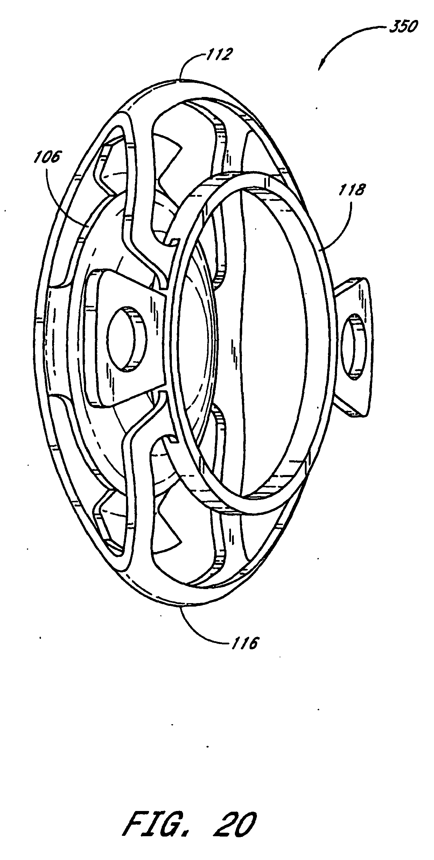

Preferably, both the anterior viewing element 106 and the posterior viewing element 118 comprise an optic or lens having refractive power. (As used herein, the term "refractive" or "refractive power" shall include "diffractive" or "diffractive power".) The preferred power ranges for the optics are discussed in detail below. In alternative embodiments one or both of the anterior and posterior viewing elements 106, 118 may comprise an optic with a surrounding or partially surrounding perimeter frame member or members, with some or all of the biasing elements/translation members attached to the frame member(s). As a further alternative, one of the viewing elements 106, 118 may comprise a perimeter frame with an open/empty central portion or void located on the optical axis (see Figure 20 and discussion below), or a perimeter frame member or members with a zero-power lens or transparent member therein. In still further variations, one of the viewing elements 106, 118 may comprise only a zero-power lens or transparent member.

In a presently preferred embodiment, a retention portion 126 is coupled to the anterior portion 102, preferably at the anterior viewing element 106. The retention portion 126 preferably includes a first retention member 128 and a second retention member 130, although in alternative embodiments the retention portion 126 may be omitted altogether, or may comprise only one retention member or more than two retention members. The first retention member 128 is coupled to the anterior viewing element 106 at a fixed end 128a and also includes a free end 128b opposite the fixed end 128a. Likewise, the second retention member 130 includes a fixed end 130a and a free end 130b. The retention members 128, 130 are illustrated as being coupled to the anterior viewing element 106 at the upper and lower edges thereof; however, the retention members 128,130 may alternatively be attached to the anterior viewing element 106 at other suitable edge locations.

In the preferred embodiment, the posterior portion 104 includes a distending portion 132, preferably attached to the posterior viewing element 118. The preferred distending portion 132 includes a first distending member 134 which in turn includes a fixed end 134a, a free end 134b opposite the fixed end 134a and preferably also includes an opening 134c formed therein. The preferred distending portion 132 also comprises a second distending member 136 with a fixed end 136a, a free end 136b and preferably an opening 136c formed therein. In alternative embodiments, the distending portion 132 may be omitted altogether, or may comprise a single distending member or more than two distending members. To optimize their effectiveness, the preferred location for the distending members 134, 136 is 90 degrees away (about the optical axis) from the apices 112, 116 on the posterior portion 104. Where the biasing elements form more than two apices (or where two apices are not spaced 180 degrees apart about the optical axis), one or more distending members may be positioned angularly midway between the apices about the optical axis. Alternatively, the distending member(s) may occupy other suitable positions relative to the apices (besides the "angularly midway" positions disclosed above); as further alternatives, the distending member(s) may be located on the anterior portion 102 of the lens system 100, or even on the apices themselves. The functions of the retention portion 126 and the distending portion 132 will be described in greater detail below.

### III. THE LENS SYSTEM: FUNCTION/OPTICS

The anterior and posterior biasing elements 108, 120 function in a springlike manner to permit the anterior viewing element 106 and posterior viewing element 118 to move relative to each other generally along the optical axis. The biasing elements 108, 120 bias the viewing elements 106, 118 apart so that the elements 106,108 separate to the accommodated position or accommodated state shown in Figure 4. Thus, in the absence of any external forces, the viewing elements are at their maximum separation along the optical axis. The viewing elements 106, 118 of the lens system 100 may be moved toward each other, in response to a ciliary muscle force of up to 2 grams, to provide an unaccommodated position by applying appropriate forces upon the anterior and posterior portions 102, 104 and/or the apices 112, 116.

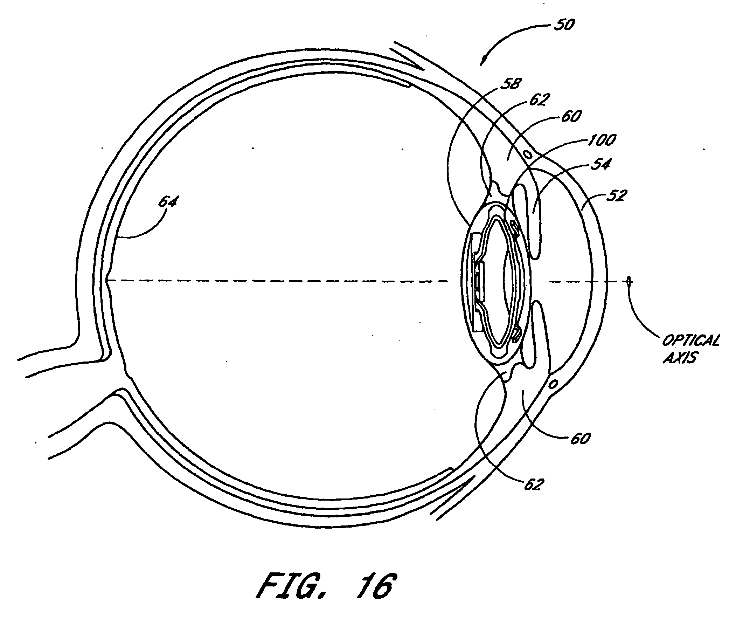

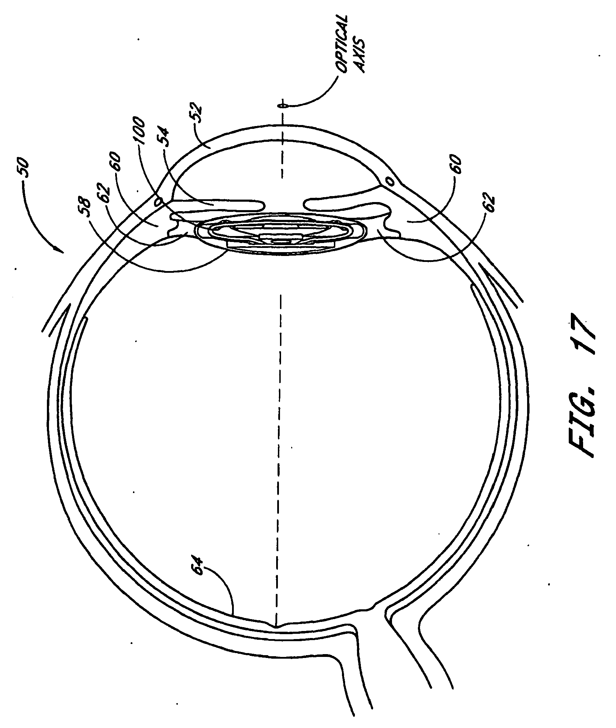

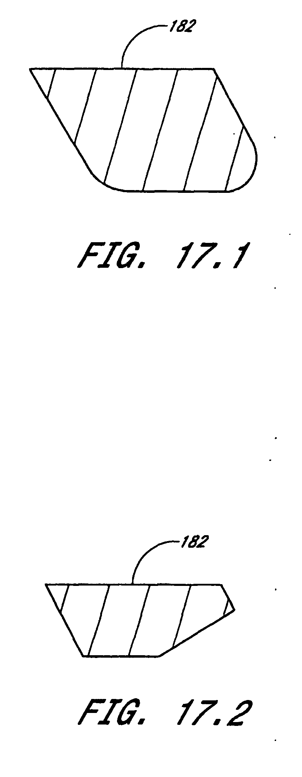

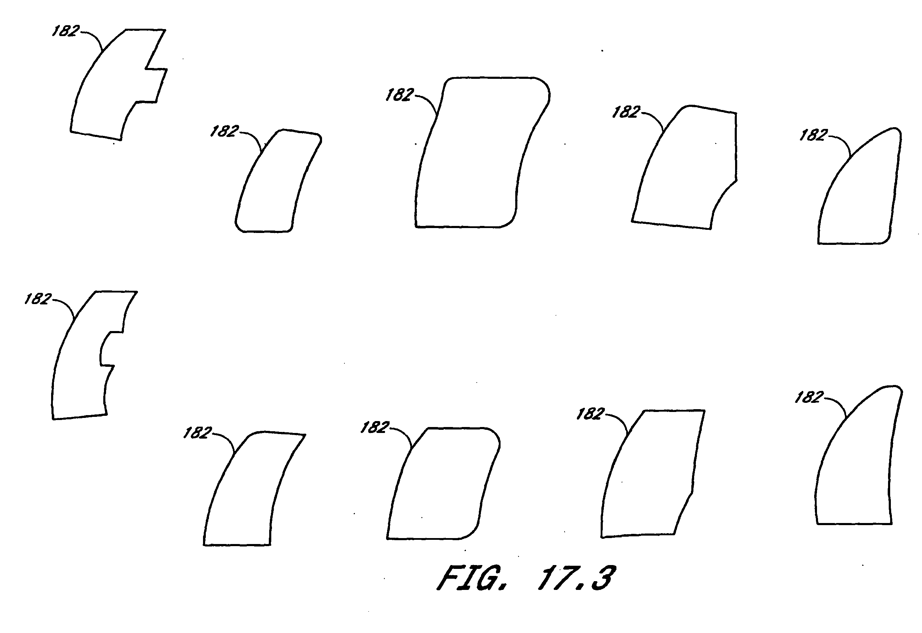

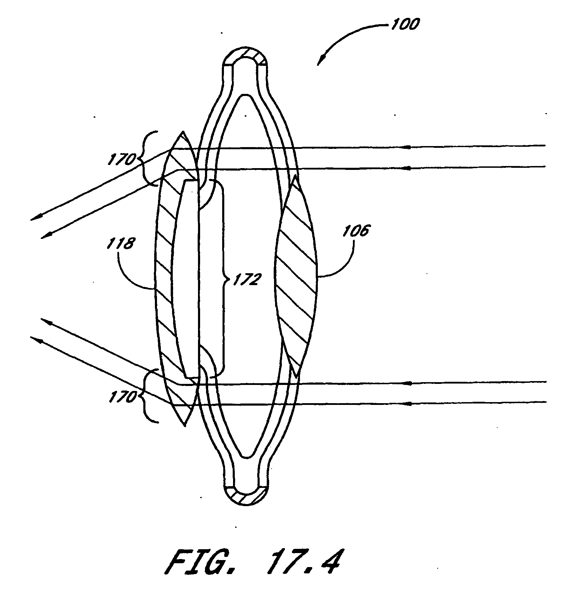

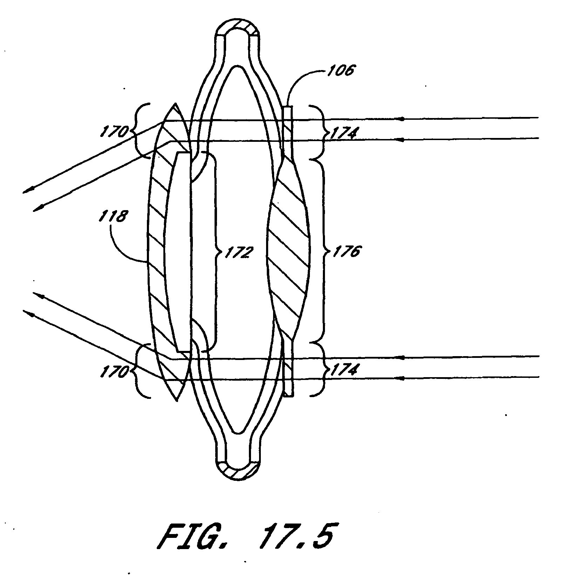

When the lens system 100 is implanted in the capsular bag 58 (Figures 16-17) the above described biasing forces cause the lens system 100 to expand along the optical axis so as to interact with both the posterior and anterior aspects of the capsular bag. Such interaction occurs throughout the entire range of motion of the ciliary muscle 60. At one extreme the ciliary muscle is relaxed and the zonules 62 pull the capsular bag 58 radially so as to cause the bag to become more disk shaped. The anterior and posterior sides of the bag, in turn, apply force to the anterior and posterior portions 102, 104 of the lens system 100, thereby forcing the viewing elements 106, 118 toward each other into the accommodated position. At the other extreme, the ciliary muscle contracts and the zonules 62 move inwardly to provide slack in the capsular bag 58 and allow the bag to become more foothall-shaped. The slack in the bag is taken up by the lens system due to the biasing-apart of the anterior and posterior viewing elements 106, 118. As the radial tension in the bag is reduced, the viewing elements 106, 118 move away from each other into an accommodated position. Thus, the distance between the viewing elements 106, 118 depends on the degree of contraction or relaxation of the ciliary muscle 60. As the distance between the anterior and posterior viewing elements 106, 118 is varied, the focal length of the lens system 100 changes accordingly. Thus, when the lens system 100 is implanted into the capsular bag (see Figures 16-17) the lens system 100 operates in conjunction with the natural accommodation processes of the eye to move between the accommodated (Figure 16) and unaccommodated (Figure 17) states in the same manner as would a healthy "natural" lens. Preferably, the lens system 100 can move between the accommodated and unaccommodated states in less than about one second.

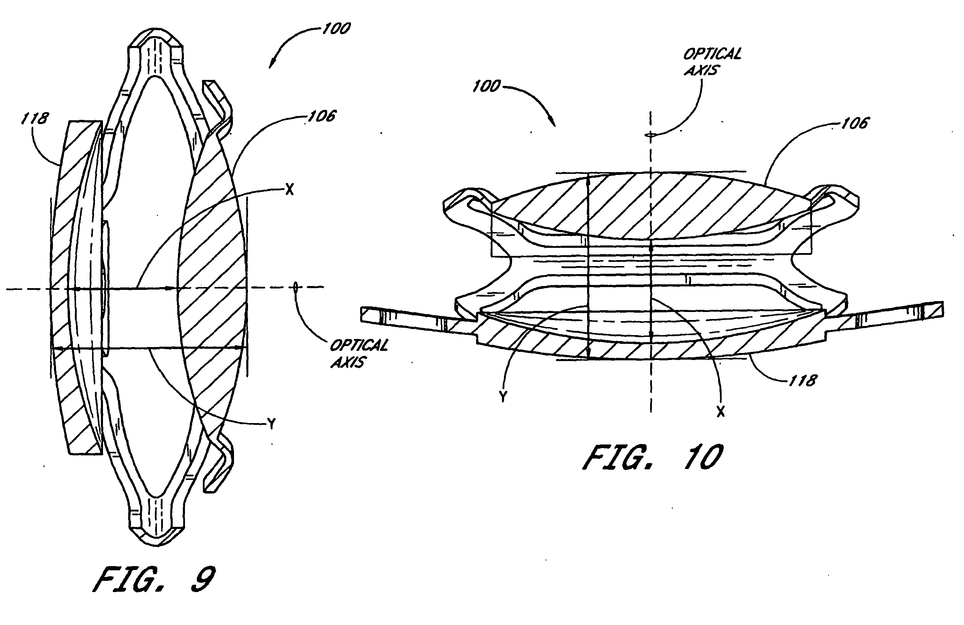

The lens system 100 has sufficient dynamic range that the anterior and posterior viewing elements 108, 118 move about 0.5-4 mm, preferably about 1-3 mm, more preferably about 1-2 mm, and most preferably about 1.5 mm closer together when the lens system 100 moves from the accommodated state to the unaccommodated state. In other words the separation distance X (see Figures 9-10, 14-15) between the anterior and posterior viewing elements 106, 118, which distance may for present purposes be defined as the distance along the optical axis (or a parallel axis) between a point of axial intersection with the posterior face of the anterior viewing element 106 and a point of axial intersection with the anterior face of the posterior viewing element 118, decreases by the amount(s) disclosed above upon movement of the lens system 100 to the unaccommodated state. Simultaneously, in the preferred mode the total system thickness Y decreases from about 3.0 - 4.0 mm in the accommodated state to about 1.5 - 2.5 mm in the unaccommodated state.

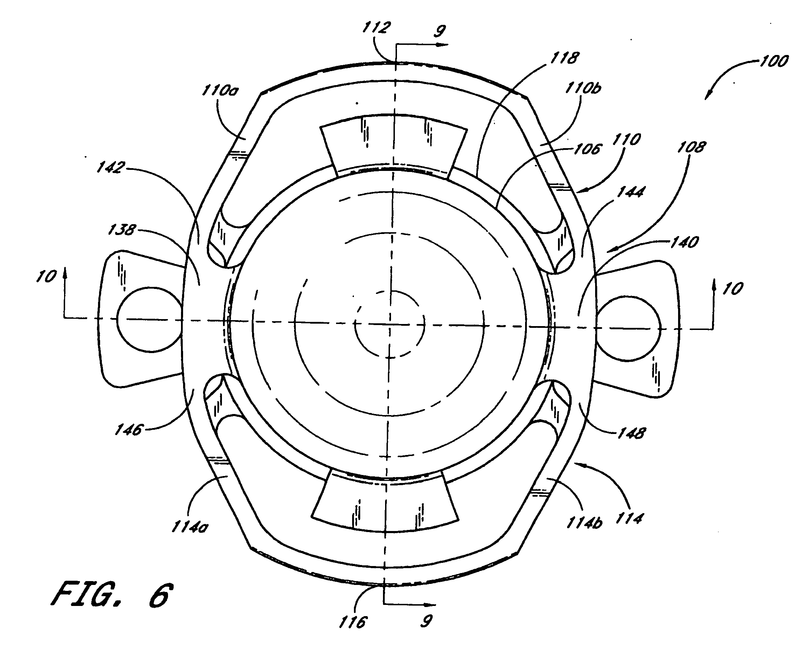

As may be best seen in Figure 6, the first anterior translation member 110 connects to the anterior viewing element 106 via connection of the left and right arms 110a, 110b to first and second transition members 138,140 at attachment locations 142, 144. The second anterior translation member 114 connects to the anterior viewing element 106 via connection of left and right arms 114a, 114b to the first and second transition members 138, 140 at attachment locations 146, 148. This is a presently preferred arrangement for the first and second anterior translation members 110, 114; alternatively, the first and second anterior translation members 110, 114 could be connected directly to the anterior viewing element 108, as is the case with the connection of the first and second posterior translation members 122, 124 to the posterior viewing element 118.

However the connection is established between the first and second anterior translation members 110, 114 and the anterior viewing element 106, it is preferred that the attachment locations 142, 144 corresponding to the first anterior translation member 110 be farther away from the first apex 112 than is the closest edge or the periphery of the anterior viewing element 106. This configuration increases the effective length of the first anterior translation member 110/arms 110a, 110b, in comparison to a direct or straight attachment between the apex 112 and the nearest/top edge of the anterior viewing element 106. For the same reasons, it is preferred that the attachment locations 146, 148 associated with the second anterior translation member 114 be farther away from the second apex 116 than is the closest/bottom edge of the anterior viewing element 106.

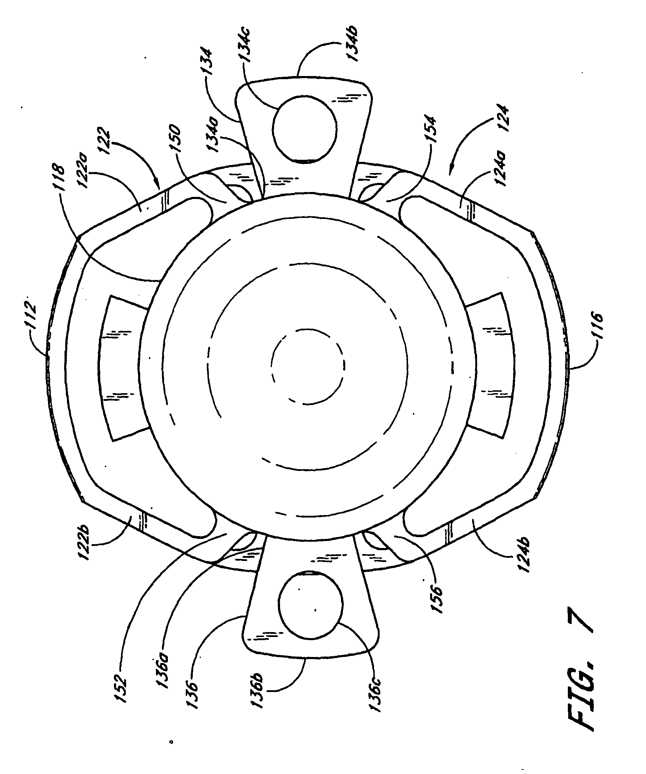

As best seen in Figure 7, the first posterior translation member 122 is preferably connected directly to the posterior viewing element 118 via attachment of the left and right arms 122a, 122b to the element 118 at attachment points 150, 152. Likewise, the second posterior translation member 124 is preferably directly connected to the posterior viewing element 118 via connection of the left and right arms 124a, 124b to the element 118 at attachment points 154, 156, respectively. In alternative embodiments, the first and second posterior translation members 124, 122 can be connected to the posterior viewing element via intervening members as is done with the anterior viewing element 106. No matter how these connections are made, it is preferred that the attachment locations 150, 152 be spaced further away from the first apex 112 than is the nearest edge or the periphery of the posterior viewing element 118. Similarly, it is preferred that the attachment locations 154, 156 be spaced further away from the second apex 116 than is the closest edge of the posterior viewing element 118.

By increasing the effective length of some or all of the translation members 110, 114, 122, 124 (and that of the arms 110a, 110b, 114a, 114b, 122a, 122b, 124a, 124b where such structure is employed), the preferred configuration of the attachment locations 142, 144, 146, 148, 150, 152, 154, 156 relative to the first and second apices 112, 116 enables the anterior and/or posterior viewing elements 106, 118 to move with respect to one another a greater distance along the optical axis, for a given angular displacement of the anterior and/or posterior translation members. This arrangement thus facilitates a more responsive spring system for the lens system 100 and minimizes material fatigue effects associated with prolonged exposure to repeated flexing.

In the illustrated embodiment, the attachment location 142 of the first anterior translation member 110 is spaced from the corresponding attachment location 146 of the second anterior translation member 114 along the periphery of the anterior viewing element, and the same relationship exists between the other pairs of attachment locations 144, 148; 150, 154; and 152,156. This arrangement advantageously broadens the support base for the anterior and posterior viewing elements 106, 118 and prevents them from twisting about an axis parallel to the lateral axis, as the viewing elements move between the accommodated and unaccommodated positions.

It is also preferred that the attachment locations 142, 144 of the first anterior translation member 110 be located equidistant from the first apex 112, and that the right and left arms 110a, 110b of the member 110 be equal in length. Furthermore, the arrangement of the attachment locations 146, 148, arms 114a, 114b and second apex preferably mirrors that recited above regarding the first anterior translation member 110, while the apices 112, 116 are preferably equidistant from the optical axis and are situated 180 degrees apart. This configuration maintains the anterior viewing element 106 orthogonal to the optical axis as the viewing element 106 moves back and forth and the anterior viewing element flexes.

For the same reasons, a like combination of equidistance and equal length is preferred for the first and second posterior translation members 122, 124 and their constituent arms 122a, 122b, 124a, 124b and attachment points 150, 152, 154, 156, with respect to the apices 112, 116. However, as shown the arms 122a, 122b, 124a, 124b need not be equal in length to their counterparts 110a, 110b, 114a, 114b in the first and second anterior translation members 110, 114.

Where any member or element connects to the periphery of the anterior or posterior viewing elements 106, 118, the member defines a connection geometry or attachment area with a connection width W and a connection thickness T (see Figure 4 and the example illustrated therein, of the connection of the second posterior translation member 124 to the posterior viewing element 118). For purposes of clarity, the connection width is defined as being measured along a direction substantially parallel to the periphery of the viewing element in question, and the connection thickness is defined as measured along a direction substantially perpendicular to the periphery of the viewing element. (The periphery itself is deemed to be oriented generally perpendicular to the optical axis as shown in Figure 4.) Preferably, no attachment area employed in the lens system 100 has a ratio of width to thickness less than 3. It has been found that such a geometry reduces distortion of the viewing element/optic due to localized forces. For the same reasons, it is also preferred that each of the translation members 110, 114, 122, 124 be connected to the periphery of the respective viewing elements at least two attachment areas, each having the preferred geometry discussed above.

Figures 17.1 and 17.2 show two preferred cross-sectional configurations which may be used along some or all of the length of the translation members and/or arms 110a, 110b, 114a, 114b, 122a, 122b, 124a, 124b. The shape is defined by a relatively broad and flat or slightly curved outer surface 182. It is intended that when in use the outer surface faces away from the interior of the lens system and/or toward the capsular bag 58. The remaining surfaces, proportions and dimensions making up the cross-sectional shape can vary widely but may advantageously be selected to facilitate manufacture of the lens system 100 via molding or casting techniques while minimizing stresses in the arms during use of the lens system.

Figure 17.3 depicts a number of alternative cross-sectional configurations which are suitable for the translation members and/or arms 110a, 110b, 114a, 114b, 122a, 122b, 124a, 124b. As shown, a wide variety of cross-sectional shapes may be used, but preferably any shape includes the relatively broad and flat or slightly curved outer surface 182.

It is further contemplated that the dimensions, shapes, and/or proportions of the cross-sectional configuration of the translation members and/or arms 110a, 110b, 114a, 114b, 122a, 122b, 124a, 124b may vary along the length of the members/arms. This may be done in order to, for example, add strength to high-stress regions of the arms, fine-tune their spring characteristics, add rigidity or flexibility, etc.

As discussed above, each of the anterior viewing element 106 and the posterior viewing element 118 preferably comprises an optic having refractive power. In one preferred embodiment, the anterior viewing element 106 comprises a biconvex lens having positive refractive power and the posterior viewing element 118 comprises a convexo-concave lens having negative refractive power. The anterior viewing element 106 may comprise a lens having a positive power advantageously less than 55 diopters, preferably less than 40 diopters, more preferably less than 35 diopters, and most preferably less than 30 diopters. The posterior viewing element 118 may comprise a lens having a power which is advantageously between -25 and 0 diopters, and preferably between -25 and -15 diopters. In other embodiments, the posterior viewing element 118 comprises a lens having a power which is between -15 and 0 diopters, preferably between -13 and -2 diopters, and most preferably between -10 and -5 diopters. Advantageously, the total power of the optic(s) employed in the lens system 100 is about 5-35 diopters; preferably, the total power is about 10-30 diopters; most preferably, the total power is about 15-25 diopters. (As used herein, the term "diopter" refers to lens or system power as measured when the lens system 100 has been implanted in the human eye in the usual manner.) It should be noted that if materials having a high index of refraction (e.g., higher than that of silicone) are used, the optics may be made thinner which facilitates a wider range of motion for the optics. This in turn allows the use of lower-power optics than those specified above. In addition, higher-index materials allow the manufacture of a higher-power lens for a given lens thickness and thereby reduce the range of motion needed to achieve a given range of accommodation.

Some lens powers and radii of curvature presently preferred for use with an embodiment of the lens system 100 with optic(s) having a refractive index of about 1.432 are as follows: a +31 diopter, biconvex lens with an anterior radius of curvature of 5.944 mm and a posterior radius of curvature of 5.944 mm; a +28 diopter, biconvex lens with an anterior radius of curvature of 5.656 mm and a posterior radius of curvature of 7.788 mm; a +24 diopter, biconvex lens with an anterior radius of curvature of 6.961 mm and a posterior radius of curvature of 8.5 mm; a -10 diopter, biconcave lens with an anterior radius of curvature of 18.765 mm and a posterior radius of curvature of 18.765 mm; a -8 diopter, concavo-convex lens with an anterior radius of curvature of between 9 mm and 9.534 mm and a posterior radius of curvature of 40 mm; and a -5 diopter, concavo-convex lens with an anterior radius of curvature of between 9 mm and 9.534 mm and a posterior radius of curvature of 20 mm. In one embodiment, the anterior viewing element comprises the +31 diopter lens described above and the posterior viewing element comprises the -10 diopter lens described above. In another embodiment, the anterior viewing element comprises the + 28 diopter lens described above and the posterior viewing element comprises the8 diopter lens described above. In another embodiment, the anterior viewing element comprises the +24 diopter lens described above and the posterior viewing element comprises the -5 diopter lens described above.

The combinations of lens powers and radii of curvature specified herein advantageously minimize image magnification. However, other designs and radii of curvature provide modified magnification when desirable.

The lenses of the anterior viewing element 106 and the posterior viewing element 118 are relatively moveable as discussed above; advantageously, this movement is sufficient to produce an accommodation of at least one diopter, preferably at least two diopters and most preferably at least three diopters. In other words, the movement of the optics relative to each other and/or to the cornea is sufficient to create a difference between (i) the refractive power of the user's eye in the accommodated state and (ii) the refractive power of the user's eye in the unaccommodated state, having a magnitude expressed in diopters as specified above. Where the lens system 100 has a single optic, the movement of the optic relative to the cornea is sufficient to create a difference in focal power as specified above.

Advantageously, the lens system 100 can be customized for an individual patient's needs by shaping or adjusting only one of the four lens faces, and thereby altering the overall optical characteristics of the system 100. This in turn facilitates easy manufacture and maintenance of an inventory of lens systems with lens powers which will fit a large population of patients, without necessitating complex adjustment procedures at the time of implantation. It is contemplated that all of the lens systems in the inventory have a standard combination of lens powers, and that a system is fitted to a particular patient by simply shaping only a designated "variable" lens face. This custom-shaping procedure can be performed to order at a central manufacturing facility or laboratory, or by a physician consulting with an individual patient. In one embodiment, the anterior face of the anterior viewing element is the designated sole variable lens face. In another embodiment, the anterior face of the posterior viewing element is the only variable face. However, any of the lens faces is suitable for such designation. The result is minimal inventory burden with respect to lens power (all of the lens systems in stock have the same lens powers) without requiring complex adjustment for individual patients (only one of the four lens faces is adjusted in the fitting process).

### IV. THE LENS SYSTEM: ALTERNATIVE EMBODIMENTS

Figure 17.4 depicts another embodiment of the lens system 100 in which the anterior viewing element 106 comprises an optic with a smaller diameter than the posterior viewing element 118, which comprises an optic with a peripheral positive-lens portion 170 surrounding a central negative portion 172. This arrangement enables the user of the lens system 100 to focus on objects at infinity, by allowing the (generally parallel) light rays incident upon the eye from an object at infinity to bypass the anterior viewing element 106. The peripheral positive-lens portion 170 of the posterior viewing element 118 can then function alone in refracting the light rays, providing the user with focused vision at infinity (in addition to the range of visual distances facilitated by the anterior and posterior viewing elements acting in concert). In another embodiment, the anterior viewing element 106 comprises an optic having a diameter of approximately 3 millimeters or less. In yet another embodiment, the anterior viewing element 106 comprises an optic having a diameter of approximately 3 millimeters or less and a refractive power of less than 55 diopters, more preferably less than 30 diopters. In still another embodiment, the peripheral positive-lens portion 170 has a refractive power of about 20 diopters.

Figure 17.5 shows an alternative arrangement in which, the anterior viewing element 106 comprises an optic having a central portion 176 with refractive power, and a surrounding peripheral region 174 having a refractive power of substantially zero, wherein the central region 176 has a diameter smaller than the optic of the posterior viewing element 118, and preferably has a diameter of less than about 3 millimeters. This embodiment also allows some incident light rays to pass the anterior viewing element (though the zero-power peripheral region 174) without refraction, allowing the peripheral positive-lens portion 170 posterior viewing element 118 to function alone as described above.

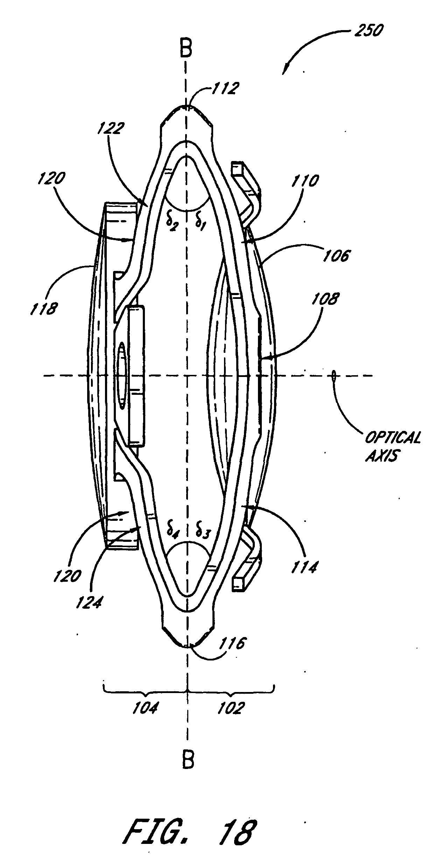

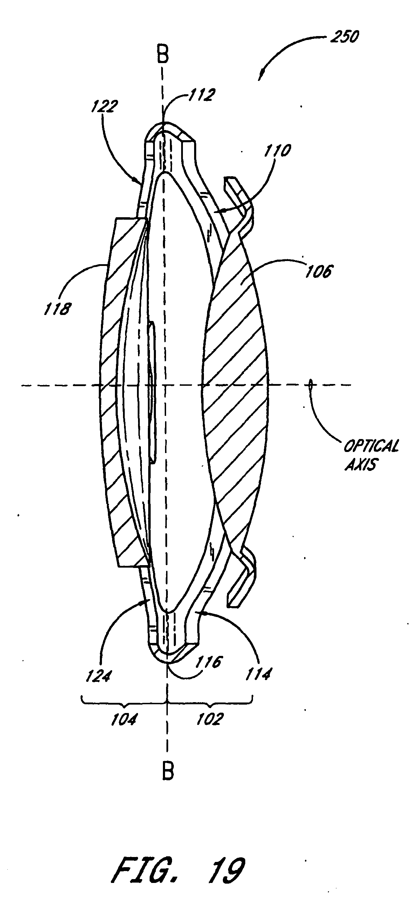

Figures 18 and 19 depict another embodiment 250 of the intraocular lens. It is contemplated that, except as noted below, this embodiment 250 is largely similar to the embodiment disclosed in Figures 3-17. The lens 250 features an anterior biasing element 108 and posterior biasing element 120 which are arranged asymmetrically as the lens system 100 is viewed from the side. As used herein to describe the biasing elements 108, 120, "asymmetric" or "asymmetrically" means that, as the lens system 100 is viewed from the side, the first anterior translation member 110 and the first posterior translation member 122 extend from the first apex 112 at unequal first anterior and posterior biasing angles 1, 2 with respect to the line B-B (which represents the edge of a plane which is substantially orthogonal to the optical axis and intersects the first and second apices 112, 116) and/or that the second anterior translation member 114 and the second posterior translation member 124 extend from the second apex 116 at substantially equal second anterior and posterior biasing angles 3, 4 with respect to the line B-B.

In the embodiment shown in Figures 18-19, the first and second anterior biasing angles 1, 3 are greater than the corresponding first and second posterior biasing angles 2, 4. This arrangement advantageously maintains the posterior viewing element 118 and apices 112, 116 in a substantially stationary position. Consequently, the moving mass of the lens system 250 is reduced, and the anterior viewing element 106 can move more quickly over a wider range along the optical axis under a given motive force. (Note that even where the posterior biasing element 120 and its constituent first and second posterior translation members 122, 124 are substantially immobile, they are nonetheless "biasing elements" and "translation members" as those terms are used herein.) In another embodiment, the anterior biasing element 108 and posterior biasing element 120 are arranged asymmetrically in the opposite direction, i.e. such that the first and second anterior biasing angles 1, 3 are smaller than the corresponding first and second posterior biasing angles 2, 4. This arrangement also provides for a wider range of relative movement of the viewing elements, in comparison to a "symmetric" system.

It should be further noted that the viewing elements 106, 118 shown in Figures 18-19 are asymmetrically positioned in that the posterior viewing element 118 is closer to the line B-B than is the anterior viewing element 106. It has been found that this configuration yields desirable performance characteristics irrespective of the configuration of the biasing elements 108, 120. In alternative embodiments, the viewing elements 106, 118 may be positioned symmetrically with respect to the line B-B, or they may be positioned asymmetrically with the anterior viewing element 106 closer to the line B-B than the posterior viewing element 118 (see Figure 4 wherein the line in question is denoted A-A). Furthermore, the symmetry or asymmetry of the biasing elements and viewing elements can be selected independently of each other.

Figure 20 shows another embodiment 350 of an intraocular lens in which the posterior viewing element 118 comprises an annular frame member defining a void therein, while the anterior viewing element 106 comprises an optic having refractive power. Alternatively, the posterior viewing element 118 could comprise a zero power lens or a simple transparent member. Likewise, in another embodiment the anterior viewing element 106 could comprise an annular frame member with a void therein or a simple zero power lens or transparent member, with the posterior viewing element 118 comprising an optic having refractive power. As a further alternative, one or both of the anterior and posterior viewing elements 106, 118 may comprise an annular or other perimeter frame member which can receive a removable optic (or a "one-time install" optic) with an interference type fit and/or subsequent adhesive or welding connections. Such a configuration facilitates assembly and/or fine-tuning of the lens system during an implantation procedure, as will be discussed in further detail below.

### V. THE LENS SYSTEM: ADDITIONAL FEATURES

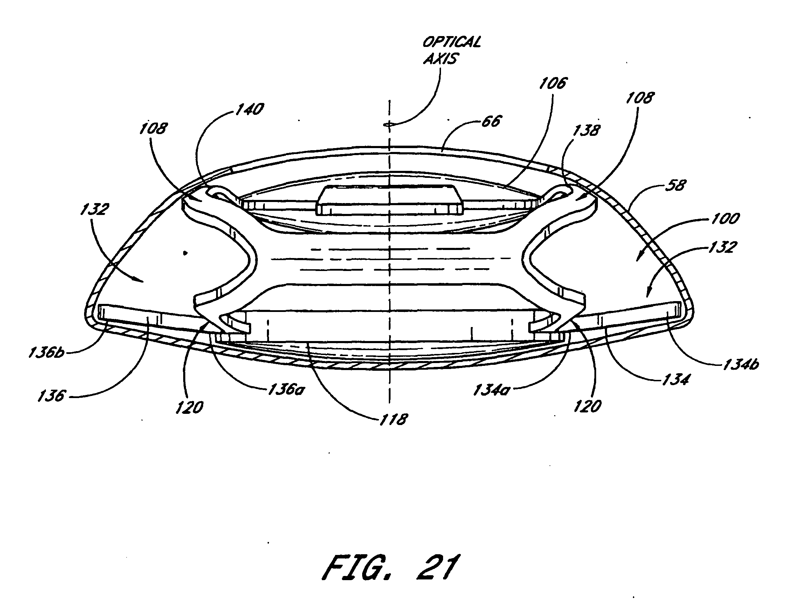

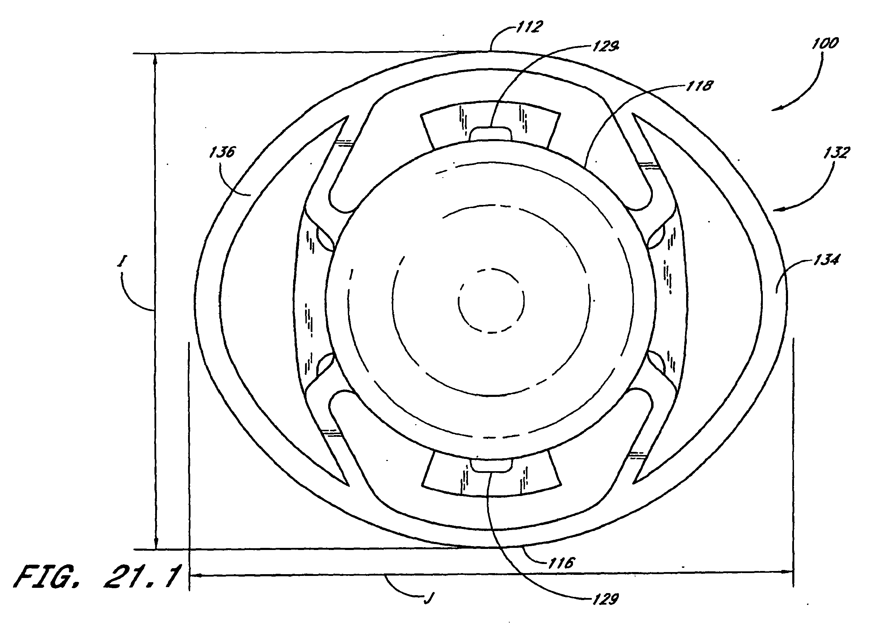

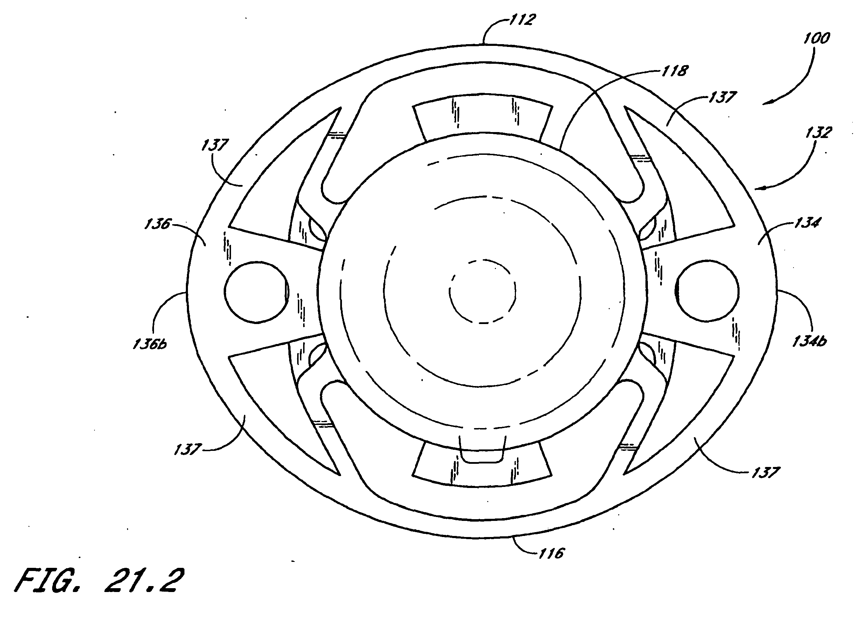

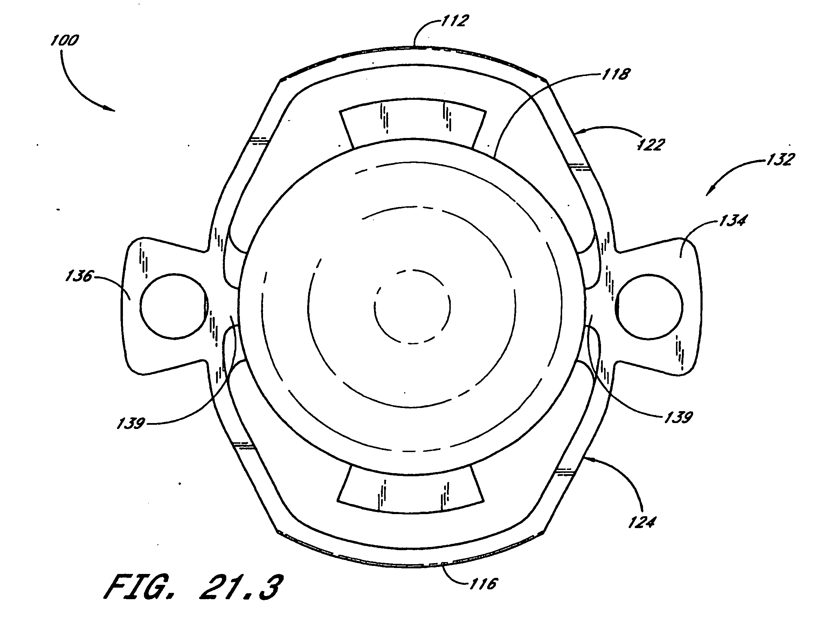

Figure 21 depicts the function of the distending portion 132 in greater detail. The lens system 100 is shown situated in the capsular bag 58 in the customary manner with the anterior viewing element 106 and posterior viewing element 118 arranged along the optical axis. The capsular bag 58 is shown with a generally circular anterior opening 66 which may often be cut into the capsular bag during installation of the lens system 100. The first and second distending members 134, 136 of the distending portion 132 distend the capsular bag 58 so that intimate contact is created between the posterior face of the posterior viewing element and/or the posterior biasing element 120. In addition, intimate contact is facilitated between the anterior face of the anterior viewing element 106 and/or anterior biasing element 108. The distending members 134, 136 thus remove any slack from the capsular bag 58 and ensure optimum force coupling between the bag 58 and the lens system 100 as the bag 58 is alternately stretched and released by the action of the ciliary muscle.

Furthermore, the distending members 134, 136 reshape the capsular bag 58 into a taller, thinner configuration along its range of accommodation to provide a wider range of relative motion of the viewing elements 106, 118. When the capsular bag 58 is in the unaccommodated state, the distending members 134, 136 force the capsular bag into a thinner configuration (as measured along the optical axis) in comparison to the unaccommodated configuration of the capsular bag 58 with the natural lens in place. Preferably, the distending members 134,136 cause the capsular bag 58 to taken on a shape in the unaccommodated state which is about 1.02.0 mm thinner, more preferably about 1.5 mm thinner, along the optical axis than it is with the natural lens in place and in the unaccommodated state.

With such a thin "starting point" provided by the distending members 134,136, the viewing elements 106, 118 of the lens system can move a greater distance apart, and provide a greater range of accommodation, without causing undesirable contact between the lens system and the iris. Accordingly, by reshaping the bag as discussed above the distending members 134, 136 facilitate a range of relative motion of the anterior and posterior viewing elements 106, 118 of about 0.5-4 mm, preferably about 1-3 mm, more preferably about 1-2 mm, and most preferably about 1.5 mm.

The distending portion 132/distending members 134, 136 are preferably separate from the anterior and posterior biasing elements 108,120; the distending members 134,136 thus preferably play no part in biasing the anterior and posterior viewing elements 106, 118 apart toward the accommodated position. This arrangement is advantageous because the apices 112, 116 of the biasing elements 108, 120 reach their point of minimum protrusion from the optical axis (and thus the biasing elements reach their minimum potential effectiveness for radially distending the capsular bag) when the lens system 100 is in the accommodated state (see Figure 16), which is precisely when the need is greatest for a taut capsular bag so as to provide immediate response to relaxation of the ciliary muscles. The preferred distending portion is "static" (as opposed to the "dynamic" biasing members 108, 120 which move while urging the viewing elements 106, 118 to the accommodated position or carrying the viewing elements to the unaccommodated position) in that its member(s) protrude a substantially constant distance from the optical axis throughout the range of motion of the viewing elements 106, 118. Although some degree of flexing may be observed in the distending members 134, 136, they are most effective when rigid. Furthermore, the thickness and/or cross-sectional profile of the distending members 134/136 may be varied over the length of the members as desired to provide a desired degree of rigidity thereto.

The distending portion 132/distending members 132, 134 advantageously reshape the capsular bag 58 by stretching the bag 58 radially away from the optical axis and causing the bag 58 to take on a thinner, taller shape throughout the range of accommodation by the eye. This reshaping is believed to facilitate a broad (as specified above) range of relative motion for the viewing elements of the lens system 100, with appropriate endpoints (derived from the total system thicknesses detailed above) to avoid the need for unacceptably thick optic(s) in the lens system.

If desired, the distending members 134, 136 may also function as haptics to stabilize and fixate the orientation of the lens system 100 within the capsular bag. The openings 134c, 136c of the preferred distending members 134,136 permit cellular ingrowth from the capsular bag upon positioning of the lens system 100 therein. Finally, other methodologies, such as a separate capsular tension ring or the use of adhesives to glue the capsular bag together in selected regions, may be used instead of or in addition to the distending portion 132, to reduce "slack" in the capsular bag.

A tension ring can also act as a physical barrier to cell growth on the inner surface of the capsular bag, and thus can provide additional benefits in limiting posterior capsule opacification, by preventing cellular growth from advancing posteriorly on the inner surface of the bag. When implanted, the tension ring firmly contacts the inner surface of the bag and defines a circumferential barrier against cell growth on the inner surface from one side of the barrier to another.

Figure 21.1 shows an alternative configuration of the distending portion 132, in which the distending members 134, 136 comprise first and second arcuate portions which connect at either end to the apices 112, 116 to form therewith an integral perimeter member. In this arrangement it is preferred that the distending members and apices form an oval with height I smaller than width J.

Figure 21.2 shows another alternative configuration of the distending portion 132, in which arcuate rim portions 137 interconnect the apices 112, 116 and the free ends 134b, 136b of the distending members 134, 136. Thus is formed an integral perimeter member with generally higher lateral rigidity than the arrangement depicted in Figure 21.1.

Figure 21.3 shows another alternative configuration of the distending portion 132, in which the distending members 134, 136 are integrally formed with the first and second posterior translation members 122, 124. The distending members 134, 136 and translation members 122, 124 thus form common transition members 139 which connect to the periphery of the posterior viewing element 118.

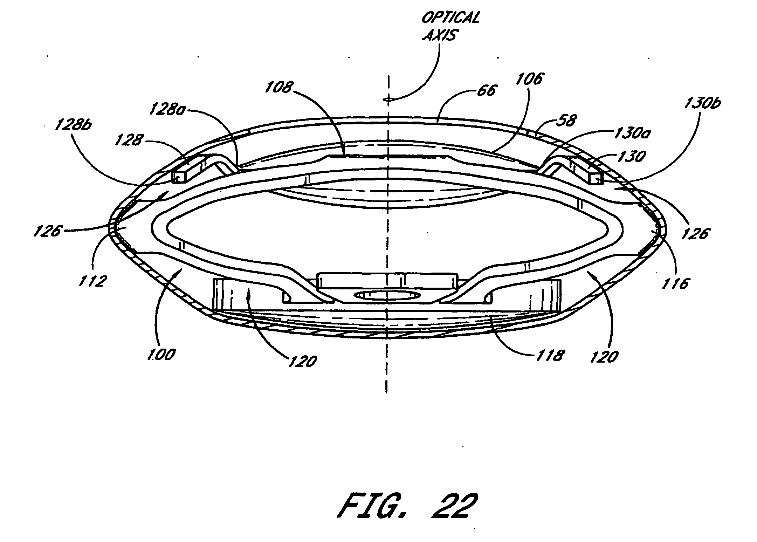

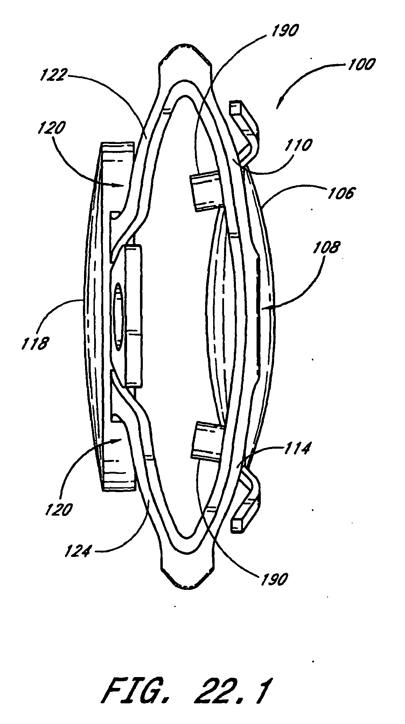

Figure 22 shows the function of the retention portion 126 in greater detail. It is readily seen that the first and second retention members 128, 130 facilitate a broad contact base between the anterior portion of the lens system 100 and the anterior aspect of the capsular bag 58. By appropriately spacing the first and second retention members 128, 130, the members prevent extrusion of the anterior viewing element 106 through the anterior opening 66. It is also readily seen that where contact occurs between the anterior aspect of the capsular bag 58 and one or both of the retention members 128, 130, the retention members also participate in force coupling between the bag 58 and the lens system 100 as the bag is stretched and released by the action of the ciliary muscles.

As best seen in Figures 21 and 22, the anterior portion 102 of the lens system 100 forms a number of regions of contact with the capsular bag 58, around the perimeter of the anterior viewing element 106. In the illustrated embodiment, at least some of these regions of contact are located on the anteriormost portions of the anterior biasing element 108, specifically at the transition members 138, 140, and at the retention members 128, 130. The transition members and the retention members define spaces therebetween at the edges of the anterior viewing element 106 to permit fluid to flow between the interior of the capsular bag 58 and the portions of the eye anterior of the bag 58. In other words, the anterior portion of the lens system 100 includes at least one location which is spaced from and out of contact with the capsular bag 58 to provide a fluid flow channel extending from the region between the viewing elements 106, 118 to the exterior of the bag 58. Otherwise, if the anterior portion 102 of the lens system 100 seals the anterior opening 66 of the bag 58, the resulting prevention of fluid flow can cause the aqueous humor in the capsular bag to stagnate, leading to a clinically adverse event, and can inhibit the movement of the lens system 100 between the accommodated and unaccommodated states.

If desired, one or both of the retention members 128, 130 may have an opening 129 formed therein to permit fluid flow as discussed above. (See Figure 21.1.)

The retention members 128, 130 and the transition members 138, 140 also prevent contact between the iris and the anterior viewing element 106, by separating the anterior opening 66 from the anterior face of the viewing element 106. In other wards, the retention members 128, 130 and the transition members 138, 140 displace the anterior aspect of the capsular bag 58, including the anterior opening 66, anteriorly from the anterior viewing element 106, and maintain this separation throughout the range of accommodation of the lens system. Thus, if contact occurs between the iris and the lens system-capsular bag assembly, no part of the lens system will touch the iris, only the capsular bag itself, in particular those portions of the bag 58 overlying the retention members 128, 130 and/or the transition members 138, 140. The retention members 128, 130 and/or the transition members 138, 140 therefore maintain a separation between the iris and the lens system, which can be clinically adverse if the contacting portion(s) of the lens system are constructed from silicone.

As depicted in Figure 22.1, one or more stop members 190 may be located where appropriate on the anterior and/or posterior biasing elements 108, 120 to limit the convergent motion of the anterior and posterior viewing elements 106, 118, and preferably prevent contact therebetween. As the lens system 100 moves toward the unaccommodated position, the stop member(s) located on the anterior biasing element 108 come into contact with the posterior biasing element 120 (or with additional stop member(s) located on thereon), and any stop member(s) located on the posterior biasing element 120 come into contact with the anterior biasing element 108 (or with additional stop member(s) located thereon). The stop members 190 thus define a point or state of maximum convergence (in other words, the unaccommodated state) of the lens system 100/ viewing elements 106, 118. Such definition advantageously assists in setting one extreme of the range of focal lengths which the lens system may take on (in those lens systems which include two or more viewing elements having refractive power) and/or one extreme of the range of motion of the lens system 100.

The stop members 190 shown in Figure 22.1 are located on the first and second anterior translation members 110, 114 of the anterior biasing element 108 and extend posteriorly therefrom. When the anterior and posterior viewing elements 106, 118 move together, one or more of the stop members 190 will contact the posterior translation member(s) 122, 124, thereby preventing further convergent motion of the viewing elements 106, 118. Of course, in other embodiments the stop member(s) 190 can be in any suitable location on the lens system 100.

### VI. MOLD TOOLING

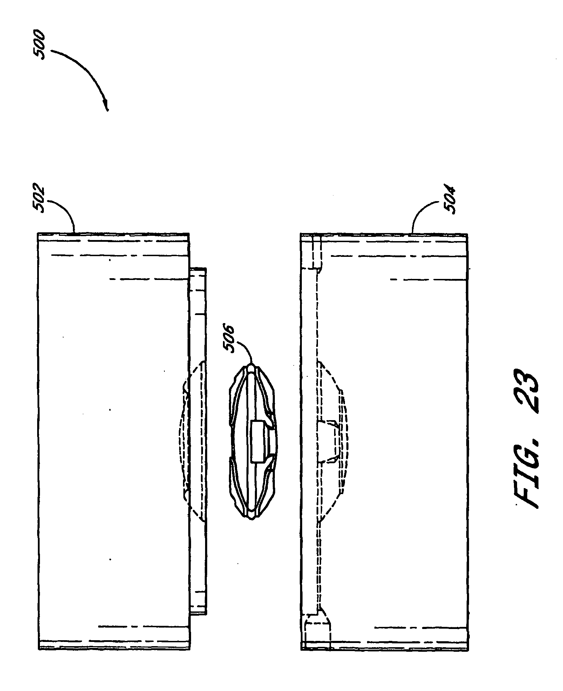

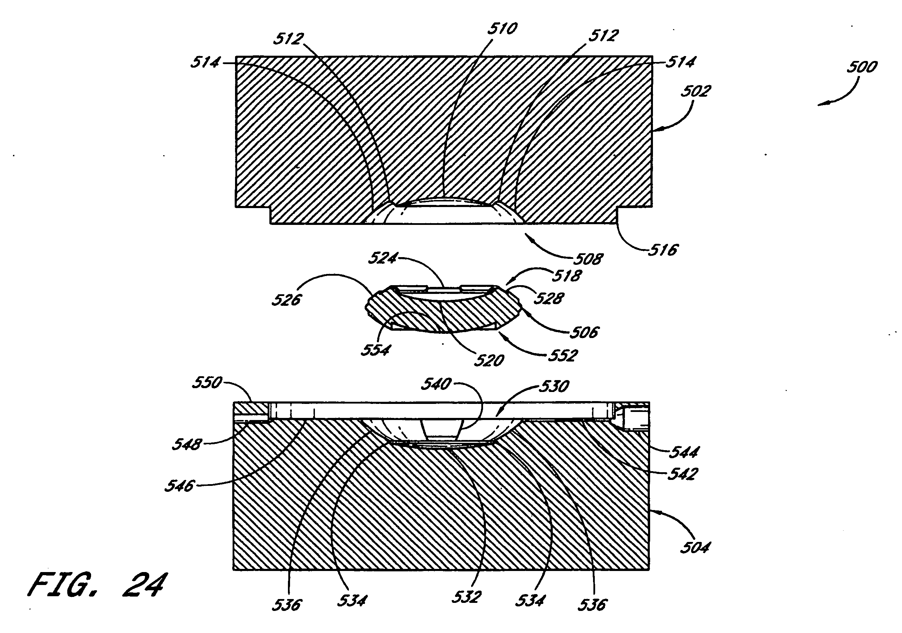

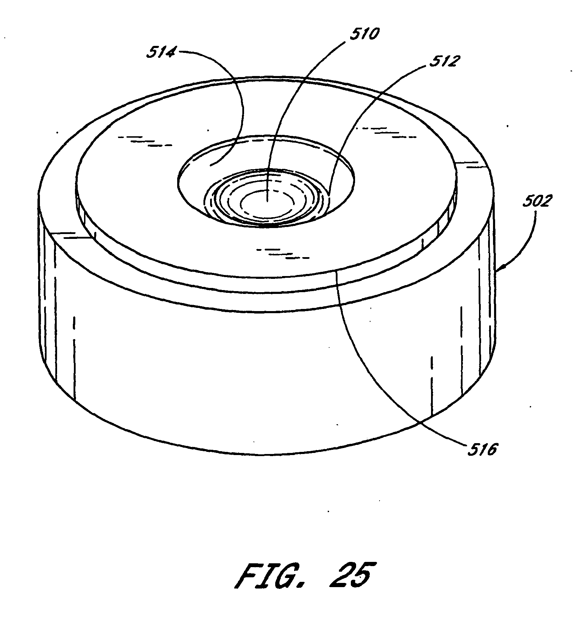

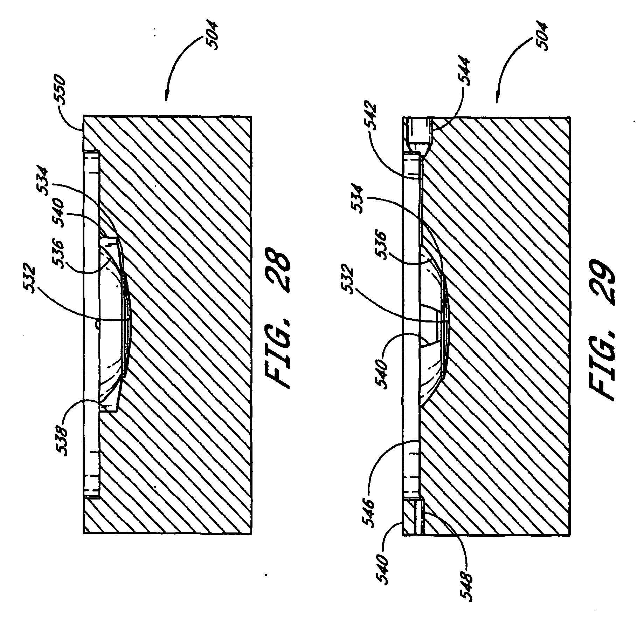

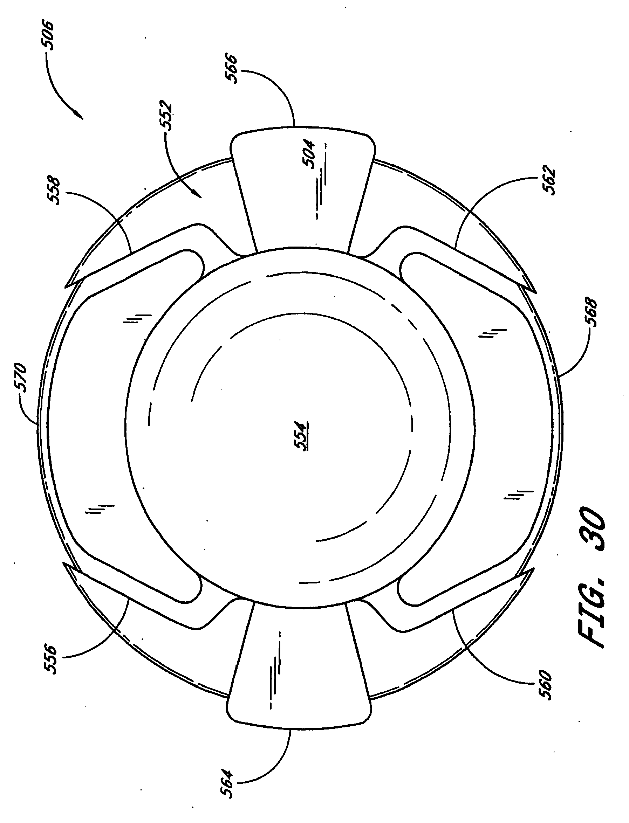

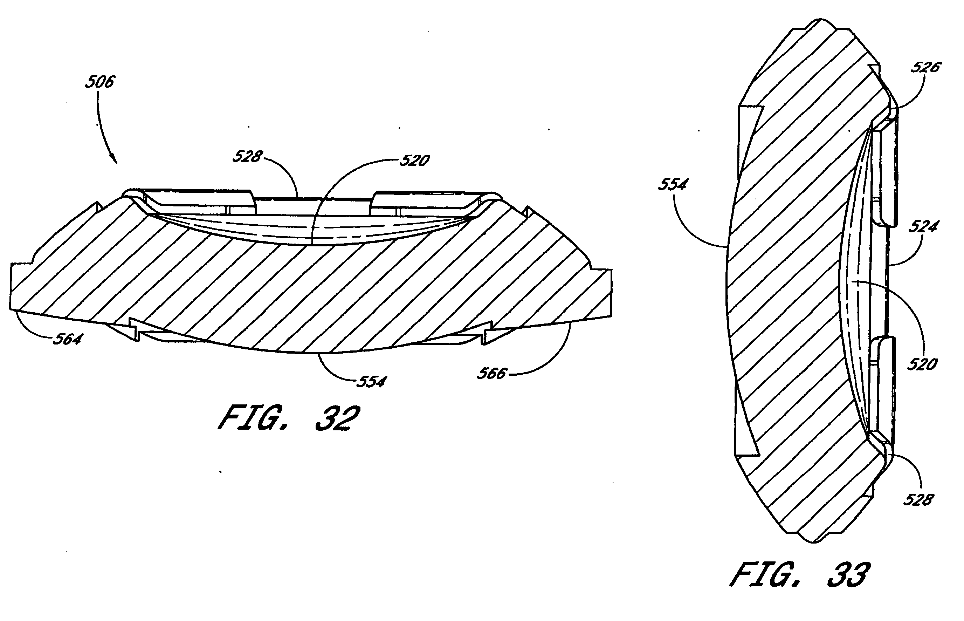

Figures 23-34 depict a mold system 500 which is suitable for molding the lens system 100 depicted in Figure 3-17. The mold system 500 generally comprises a first mold 502, a second mold 504 and a center mold 506. The center mold 506 is adapted to be positioned between the first mold 502 and the second mold 504 so as to define a mold space for injection molding or compression molding the lens system 100. The mold system 500 may be formed from suitable metals, high-impact-resistant plastics or a combination thereof, and can be produced by conventional machining techniques such as lathing or milling, or by laser or electrical-discharge machining. The mold surfaces can be finished or modified by sand blasting, etching or other texturing techniques.

The first mold 502 includes a first mold cavity 508 with a first anterior mold face 510 surrounded by an annular trough 512 and a first perimeter mold face 514. The first mold 502 also includes a projection 516 which facilitates easier mating with the second mold 504.

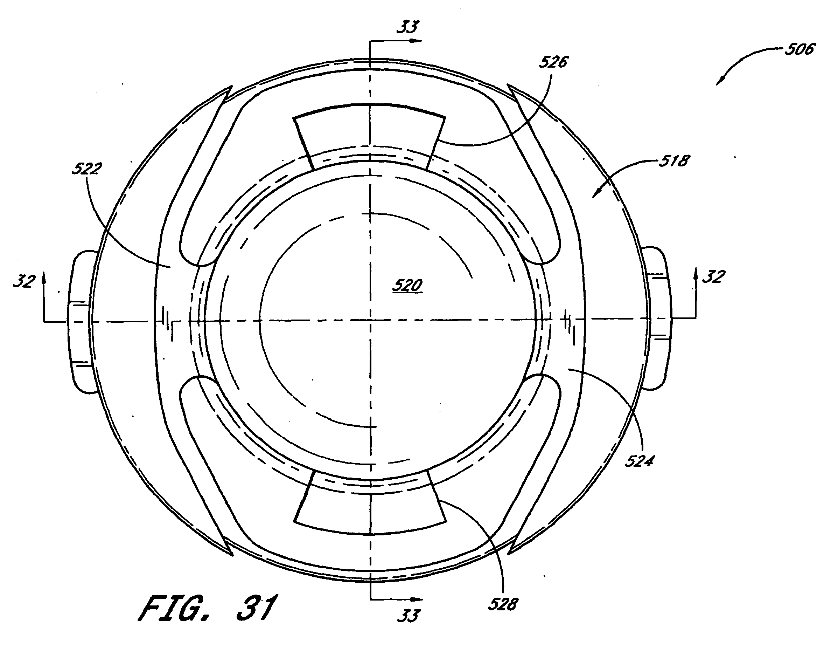

The center mold 506 includes a first center mold cavity 518 which cooperates with the first mold cavity 508 to define a mold space for forming the anterior portion 102 of the lens system 100. The first center mold cavity 518 includes a central anterior mold face 520 which, upon placement of the center mold 506 in the first mold cavity 508, cooperates with the first anterior mold face 510 to define a mold space for the anterior viewing element 106. In so doing, the first anterior mold face 510 defines the anterior face of the anterior viewing element 106 and the central anterior mold face 520 defines the posterior face of the anterior viewing element 106. In fluid communication with the chamber formed by the first anterior mold face 510 and the central anterior mold face 520 are lateral channels 522, 524 (best seen in Figure 31) which form spaces for molding the first and second transition members 138, 140, along with the arms 110a, 110b of the first anterior translation member 110 as well as the arms 114a, 114b of the second anterior translation member 114. The first center mold cavity 518 also includes retention member cavities 526, 528 which define spaces for molding the first and second retention members 128, 130 to the anterior viewing element 106.

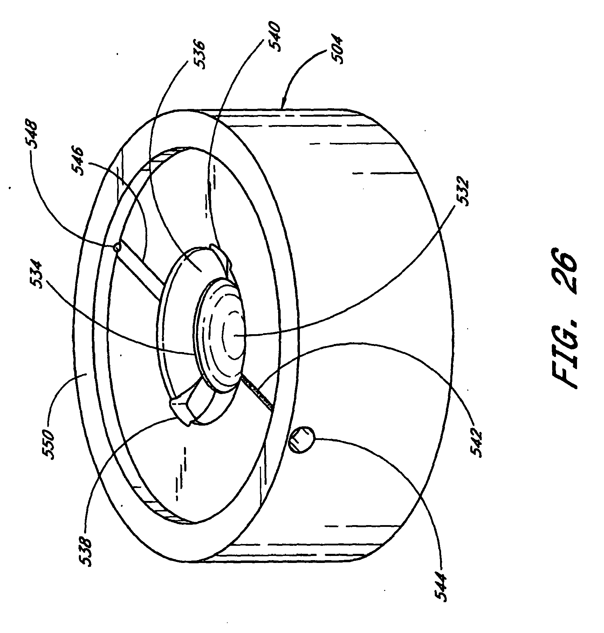

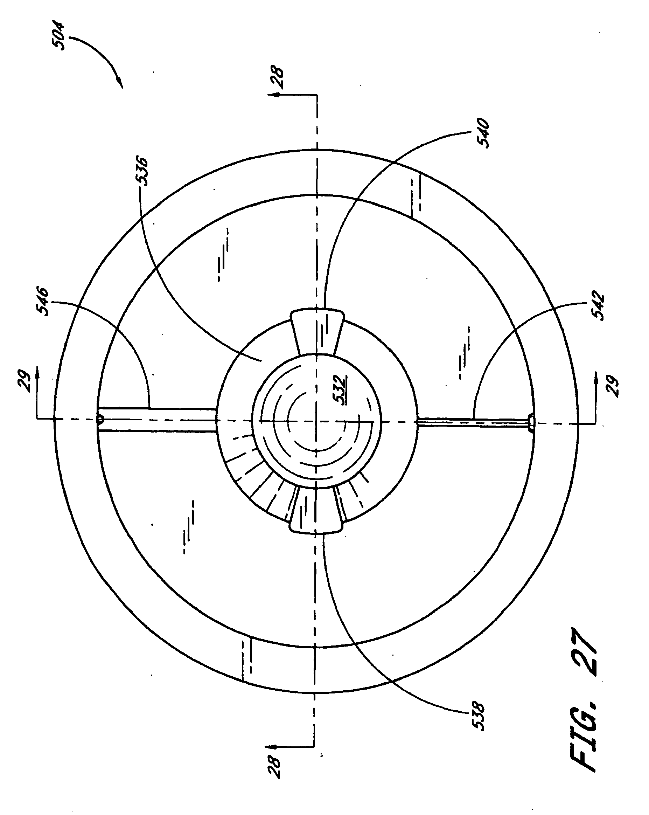

The second mold 504 includes a second mold cavity 530 with a second posterior mold space 532, a generally cylindrical transition 534 extending therefrom and connecting to a second perimeter mold face 536. Lateral notches 538, 540 (best seen in Figures 26 and 27) are formed in the second perimeter mold face 536. The second mold 504 also includes an input channel 542 connected to an input channel opening 544 for introducing material into the mold system 500. Also formed in the second mold 504 is an output channel 546 and an output channel opening 548. A generally cylindrical rim 550 is included for mating with the projection 516 of the first mold 502.

The center mold 506 includes a second center mold cavity 552 which cooperates with the second mold cavity 530 to define a mold space for the posterior portion 104 of the lens system 100. The second center mold cavity 552 includes a central posterior mold face 554 which, upon placement of the center mold 506 in engagement with the second mold cavity 530, cooperates with the second posterior mold face 532 and the transition 534 to define a chamber for forming the posterior viewing element 118. In fluid communication with the chamber formed by the central posterior mold face 554 and the second posterior mold face 532 are lateral channels 556, 558, 580, 562 which provide a mold space for forming the arms 122a, 122b of the first posterior translation member 122 and the arms 124a, 124b of the second posterior translation member 124. The second center mold cavity 552 includes lateral projections 564, 566 which coact with the notches 538, 540 formed in the second mold cavity 530. The chambers formed therebetween are in fluid communication with the chamber defined by the central posterior mold face 554 and the second posterior mold face 532 to form the first and second distending members 134, 136 integrally with the posterior viewing element 118.

The center mold 506 includes a first reduced-diameter portion 568 and a second reduced-diameter portion 570 each of which, upon assembly of the mold system 500, defines a mold space for the apices 112, 116 of the lens system 100.

In use, the mold system 500 is assembled with the center mold 506 positioned between the first mold 502 and the second mold 504. Once placed in this configuration, the mold system 500 is held together under force by appropriate techniques, and lens material is introduced into the mold system 500 via the input channel 542. The lens material then fills the space defined by the first mold 502, second mold 504, and the center mold 506 to take on the shape of the finished lens system 100.

In another embodiment, the lens system 100 or a portion thereof is formed by a casting or liquid-casting procedure in which one of the first or second molds is first filled with a liquid and the center mold is placed then into engagement with the liquid-filled mold. The exposed face of the center mold is then filled with liquid and the other of the first and second molds is placed into engagement with the rest of the mold system. The liquid is allowed or caused to set/cure and a finished casting may then removed from the mold system.

The mold system 500 can advantageously be employed to produce a lens system 100 as a single, integral unit. Alternatively, various portions of the lens system 100 can be separately molded, casted, machined, etc. and subsequently assembled to create a finished lens system. Assembly can be performed as a part of centralized manufacturing operations; alternatively, a physician can perform some or all of the assembly before or during the implantation procedure, to select lens powers, biasing members, system sizes, etc. which are appropriate for a particular patient.

The center mold 506 is depicted as comprising an integral unit with first and second center mold cavities 518, 552. Alternatively, the center mold 506 may have a modular configuration whereby the first and second mold cavities 518, 552 may be interchangeable to adapt the center mold 506 for manufacturing a lens system 100 according to a desired prescription or specification, or to otherwise change the power(s) of the lenses made with the mold. In this manner the manufacture of a wide variety of prescriptions may be facilitated by a set of mold cavities which can be assembled back-to-back or to opposing sides of a main mold structure.

### VII. MATERIALS/SURFACE TREATMENTS

Preferred materials for forming the lens system 100 include silicone, acrylics, polymethylmethacrylate (PMMA), block copolymers of styrene-ethyfene-butylene-styrene (C-FLEX) or other styrene-base copolymers, polyvinyl alcohol (PVA), polyurethanes, hydrogels or any other suitable polymers or monomers. In addition, any portion of the lens system 100 other than the optic(s) may be formed from stainless steel or a shape-memory alloy such as nitinol or any iron-based shape-memory alloy. Metallic components may be coated with gold to increase biocompatibility. Where feasible, material of a lower Shore A hardness such as 15A may be used for the optic(s), and material of higher hardness such as 35A may be used for the balance of the lens system 100. Finally, the optic(s) may be formed from a photosensitive silicone to facilitate post-implantation power adjustment as taught in U.S. Patent Application Serial No. 09/416,044, filed October 8, 1999, titled LENSES CAPABLE OF POST-FABRICATION POWER MODIFICATION, the entire contents of which are hereby incorporated by reference herein.

Methyl-methylacrylate monomers may also be blended with any of the non-metallic materials discussed above, to increase the lubricity of the resulting lens system (making the lens system easier to fold or roll for insertion, as discussed further below). The addition of methyl-methylacrylate monomers also increases the strength and transparency of the lens system.

The optics and/or the balance of the lens system 100 can also be formed from layers of differing materials. The layers may be arranged in a simple sandwich fashion, or concentrically. In addition, the layers may include a series of polymer layers, a mix of polymer and metallic layers, or a mix of polymer and monomer layers. In particular, a nitinol ribbon core with a surrounding silicone jacket may be used for any portion of the lens system 100 except for the optics; an acrylic-over-silicone laminate may be employed for the optics. A layered construction may be obtained by pressing/bonding two or more layers together, or deposition or coating processes may be employed.

In one embodiment, portions of the lens system 100 other than the optic(s) are formed from a shape-memory alloy. This embodiment takes advantage of the exceptional mechanical properties of shape-memory alloys and provides fast, consistent, highly responsive movement of the optic(s) within the capsular bag while minimizing material fatigue in the lens system 100. In one embodiment, one or both of the biasing elements 108, 120 are formed from a shape-memory alloy such as nitinol or any iron-based shape-memory alloy. Due to the flat stress-strain curve of nitinol, such biasing elements provide a highly consistent accommodation force over a wide range of displacement. Furthermore, biasing elements formed from a shape-memory alloy, especially nitinol, retain their spring properties when exposed to heat (as occurs upon implantation into a human eye) while polymeric biasing elements tend to lose their spring properties, thus detracting from the responsiveness of the lens system. For similar reasons, it is advantageous to use shape-memory alloys such as those discussed above in forming any portion of a conventional (non-accommodating) intraocular lens, other than the optic.

Where desired, various coatings are suitable for components of the lens system 100. A heparin coating may be applied to appropriate locations on the lens system 100 to prevent inflammatory cell attachment (ICA) and/or posterior capsule opacification (PCO); naturally, possible locations for such a coating include the posterior biasing element 120 and the posterior face of the posterior viewing element 118. Coatings can also be applied to the lens system 100 to improve biocompatibility; such coatings include "active" coatings like P-15 peptides or RGD peptides, and "passive" coatings such as heparin and other mucopolysaccharides, collagen, fibronectin and laminin. Other coatings, including hirudin, teflon, teflon-like coatings, PVDF, fluorinated polymers, and other coatings which are inert relative to the capsular bag may be employed to increase lubricity at locations (such as the optics and distending members) on the lens system which contact the bag, or Hema or silicone can be used to impart hydrophilic or hydrophobic properties to the lens system 100.

It is also desirable subject the lens system 100 and/or the mold surfaces to a surface passivation process to improve biocompatibility. This may be done via conventional techniques such as chemical etching or plasma treatment.

Furthermore, appropriate surfaces (such as the outer edges/surfaces of the viewing elements, biasing elements, distending members, retention members, etc.) of the lens system 100 can be textured or roughened to improve adhesion to the capsular bag. This may be accomplished by using conventional procedures such as plasma treatment, etching, dipping, vapor deposition, mold surface modification, etc. As a further means of preventing ICA/PCO, a posteriorly-extending perimeter wall (not shown) may be added to the posterior viewing element 118 so as to surround the posterior face of the posterior optic. The wall firmly engages the posterior aspect of the capsular bag and acts as a physical barrier to the progress of cellular ingrowth occurring on the interior surface of the capsular bag. Finally, the relatively thick cross-section of the preferred anterior viewing element 118 (see Figures 9,10) ensures that it will firmly abut the posterior capsule with no localized flexing. Thus, with its relatively sharp rim, the posterior face of the preferred posterior viewing element 118 can itself serve as a barrier to cellular ingrowth and ICA/PCO. In order to achieve this effect, the posterior viewing element 118 is preferably made thicker than conventional intraocular lenses. As an alternative or supplement to a thick posterior viewing element, cell growth may be inhibited by forming a pronounced, posteriorly-extending perimeter rim on the posterior face of the posterior viewing element 118. Upon implantation of the lens system 100, the rim firmly abuts the inner surface of the capsular bag 58 and acts as a physical barrier to cell growth between the posterior face of the posterior viewing element 118 and the capsular bag 58.

The selected material and lens configuration should be able to withstand secondary operations after molding/casting such as polishing, cleaning and sterilization processes involving the use of an autoclave, or ethylene oxide or radiation. After the mold is opened, the lens should undergo deflashing, polishing and cleaning operations, which typically involve a chemical or mechanical process, or a combination thereof. Suitable mechanical processes include tumbling, shaking and vibration; a tumbling process may involve the use of a barrel with varying grades of glass beads, fluids such as alcohol or water and polishing compounds such as aluminum oxides. Process rates are material dependent; for example, a tumbling process for silicone should utilize a 6" diameter barrel moving at 30-100 RPM. It is contemplated that several different steps of polishing and cleaning may be employed before the final surface quality is achieved.

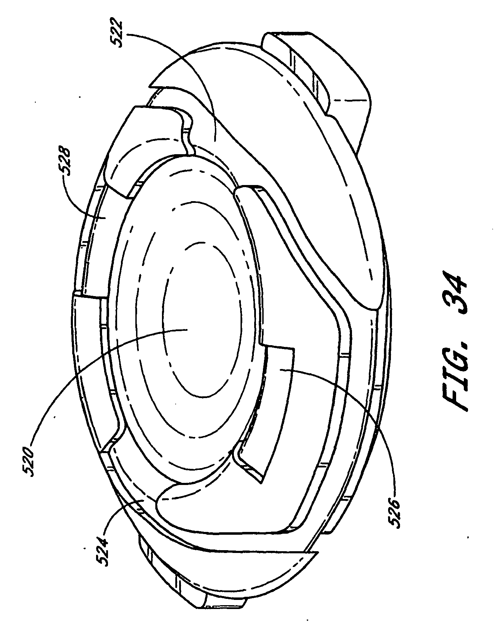

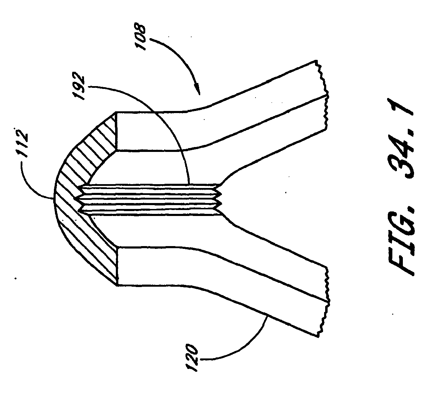

In one embodiment, the lens system 100 is held in a fixture to provide increased separation between, and improved process effect on, the anterior and posterior viewing elements during the deflashing/polishing/cleaning operations. In another embodiment, the lens system 100 is everted or turned "inside-out" so that the inner faces of the viewing elements are better exposed during a portion of the deflashing/polishing/cleaning. Figure 34.1 shows a number of expansion grooves 192 which may be formed in the underside of the apices 112, 116 of the lens system 100 to facilitate eversion of the lens system 100 without damaging or tearing the apices or the anterior/posterior biasing elements 108, 120. For the same reasons similar expansion grooves may be formed on the opposite sides (i.e, the outer surfaces) of the apices 112, 116 instead of or in addition to the location of grooves on the underside.

A curing process may also be desirable in manufacturing the lens system 100. If the lens system is produced from silicone entirely at room temperature, the curing time can be as long as several days. If the mold is maintained at about 50 degrees C, the curing time is reduced to about 24 hours; if the mold is preheated to 100-200 degrees C the curing time can be as short as about 3-15 minutes. Of course, the time-temperature combinations vary for other materials.

### VIII. MULTIPLE-PIECE AND OTHER EMBODIMENTS

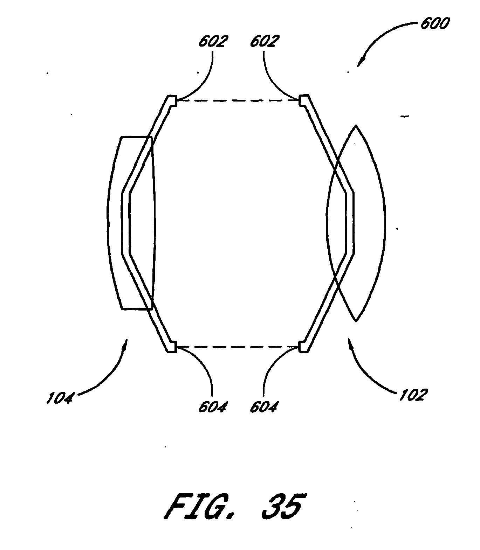

Figure 35 is a schematic view of a two-piece embodiment 600 of the lens system. In this embodiment the anterior portion 102 and the posterior portion 104 are formed as separate pieces which are intended for separate insertion into the capsular bag and subsequent assembly therein. In one embodiment, each of the anterior and posterior portions 102, 104 is rolled or folded before insertion into the capsular bag. (The insertion procedure is discussed in further detail below.) The anterior portion 102 and posterior portion 104 are represented schematically as they may generally comprise any anterior-portion or posterior-portion structure disclosed herein; for example, they may simply comprise the lens system 100 shown in Figures 3-17, bisected along the line/plane A-A shown in Figure 4. The anterior portion 102 and posterior portion 104 of the two-piece lens system 600 will include first and second abutments 602, 604 which are intended to be placed in abutting relation (thus forming the first and second apices of the lens system) during the assembly procedure. The first and second abutments 602, 604 may include engagement members (not shown), such as matching projections and recesses, to facilitate alignment and assembly of the anterior and posterior portions 102, 104.

As a further alternative, the anterior and posterior portions 102, 104 of the lens system 600 may be hingedly connected at one of the abutments 602, 604 and unconnected at the other, to allow sequential (but nonetheless partially assembled) insertion of the portions 102, 104 into the capsular bag. The individual portions may be separately rolled or folded before insertion. The two portions 102, 104 are "swung" together and joined at the unconnected abutment to form the finished lens system after both portions have been inserted and allowed to unfold/unroll as needed.

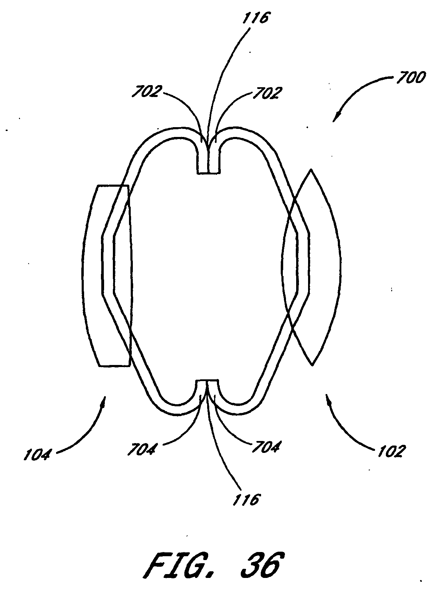

Figure 36 depicts schematically another embodiment 700 of a two-piece lens system. The lens system 700 is desirably similar to the lens system 600 shown in Figure 35, except for the formation of relatively larger, curled abutments 702, 704 which are assembled to form the apices 112, 116 of the system 700.

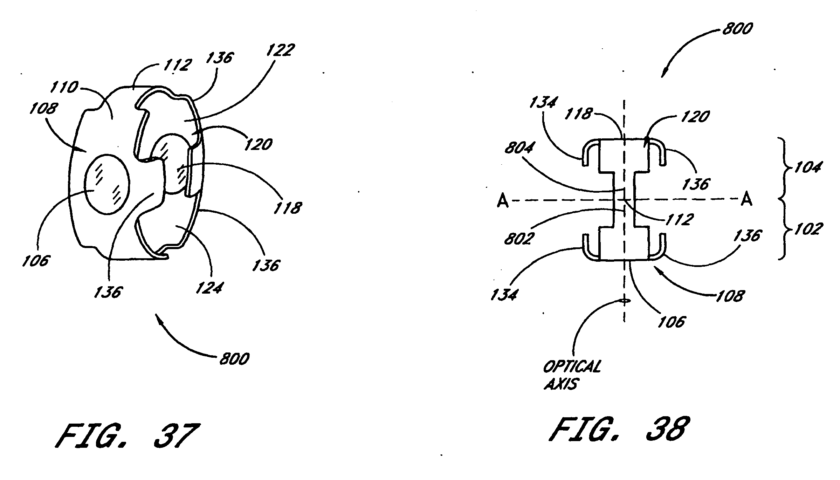

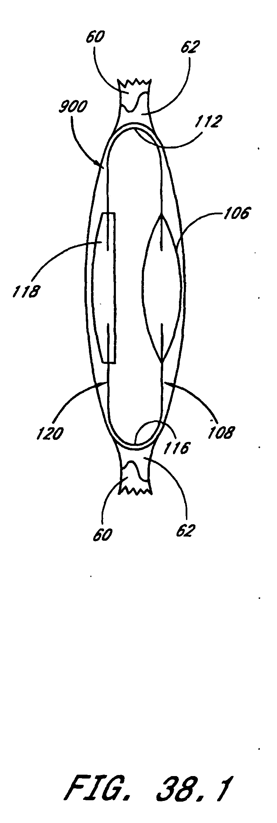

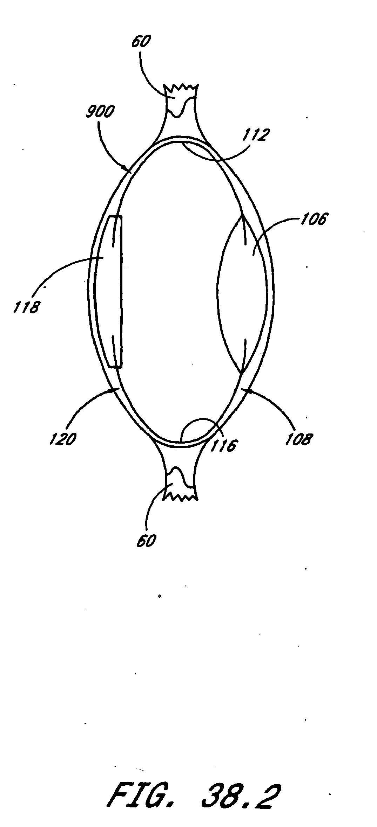

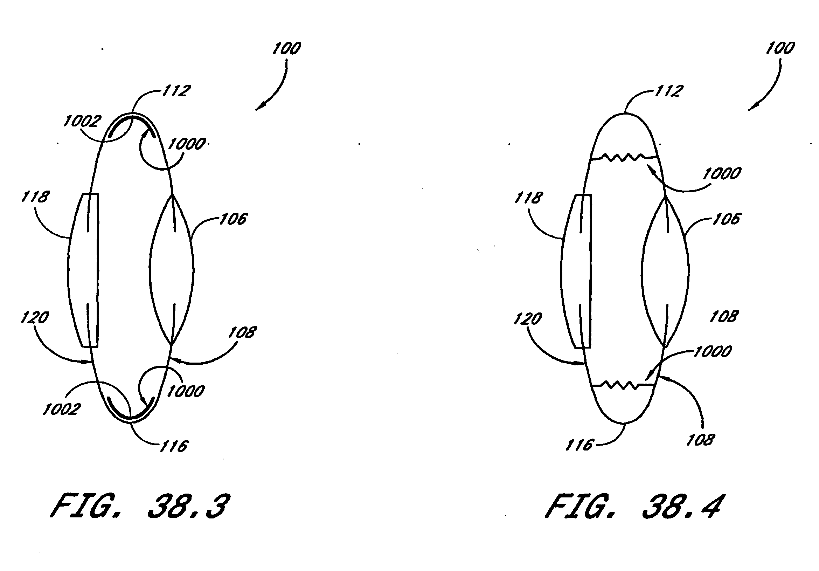

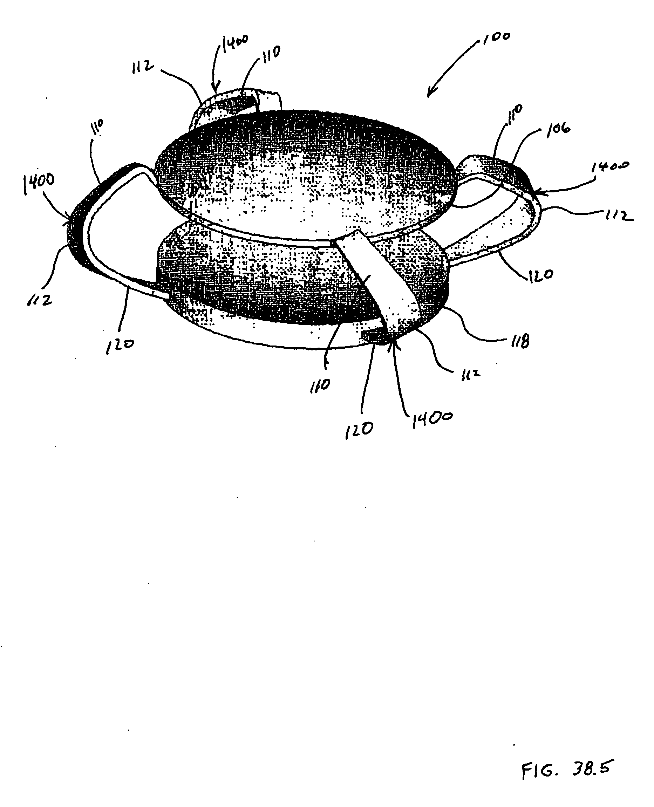

Figures 37 and 38 show a further embodiment 800 of the lens system, in which the anterior and posterior biasing elements 108, 120 comprise integral "band" like members forming, respectively, the first and second anterior translation members 110, 114 and the first and second posterior translation members 122, 124. The biasing elements 108, 120 also form reduced-width portions 802, 804 which meet at the apices of the lens system 800 and provide regions of high flexibility to facilitate sufficient accommodative movement. The depicted distending portion 132 includes three pairs of distending members 134, 136 which have a curved configuration but nonetheless project generally away from the optical axis.

Figures 38.1 and 38.2 depict another embodiment 900 of the lens system, as implanted in the capsular bag 58. The embodiment shown in Figures 38.1 and 38.2 may be similar to any of the embodiments described above, except that the biasing elements 108, 120 are dimensioned so that the apices 112, 116 abut the zonules 62 and ciliary muscles 60 when in the unaccommodated state as seen in Figure 38.1. In addition, the lens system 900 is configured such that it will remain in the unaccommodated state in the absence of external forces. Thus, when the ciliary muscles 60 contract, the muscles 60 push the apices 112, 116 closer together, causing the biasing elements 108, 120 to bow out and the viewing elements 106, 118 to separate and attain the accommodated state as shown in Figure 38.2. When the ciliary muscles 60 relax and reduce/eliminate the force applied to the apices 112, 116 the biasing elements 108, 120 move the lens system 900 to the unaccommodated state depicted in Figure 38.1.

Figures 38.3 and 38.4 depict biasers 1000 which may be used bias the lens system 100 toward the accommodated or unaccommodated state, depending on the desired operating characteristics of the lens system. It is therefore contemplated that the biasers 1000 may be used with any of the embodiments of the lens system 100 disclosed herein. The bias provided by the biasers 1000 may be employed instead of, or in addition to, any bias generated by the biasing elements 108, 120. In one embodiment (see Figure 38.3), the biasers 1000 may comprise U-shaped spring members having apices 1002 located adjacent the apices 112, 116 of the lens system 100. In another embodiment (see Figure 38.4), the biasers 1000 may comprise any suitable longitudinal-compression springs which span the apices 112, 116 and interconnect the anterior and posterior biasing elements 108, 120. By appropriately selecting the spring constants and dimensions of the biasers 1000 (in the case of Ushaped springs, the apex angle and arm length; in the case of longitudinal-compression springs, their overall length), the biasers 1000 can impart to the lens system 100 a bias toward the accommodated or unaccommodated state as desired.

The biasers 1000 may be formed from any of the materials disclosed herein as suitable for constructing the lens system 100 itself. The material(s) selected for the biasers 1000 may be the same as, or different from, the material(s) which are used to form the remainder of the particular lens system 100 to which the biasers 1000 are connected. The number of biasers 1000 used in a particular lens system 100 may be equal to or less than the number of apices formed by the biasing elements of the lens system 100.

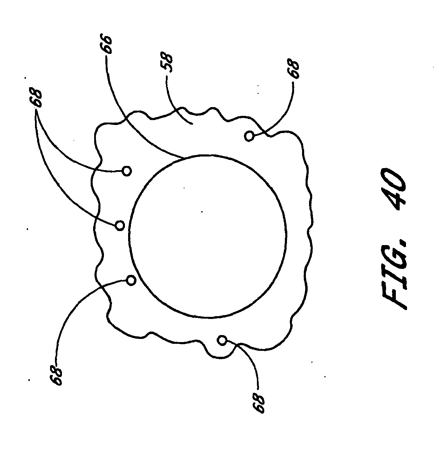

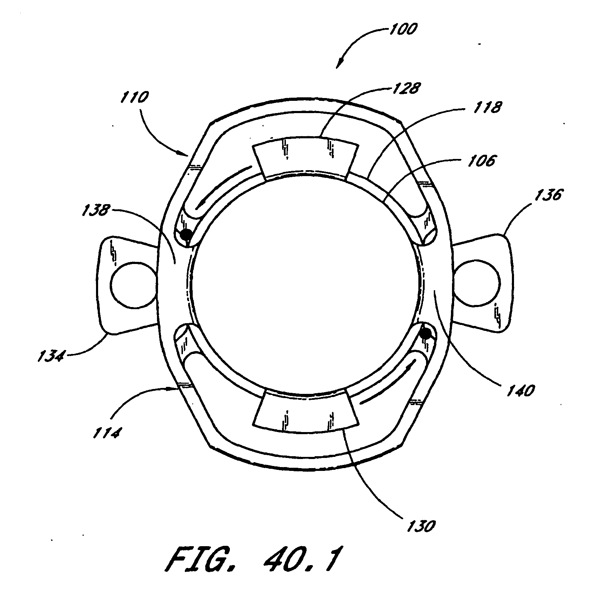

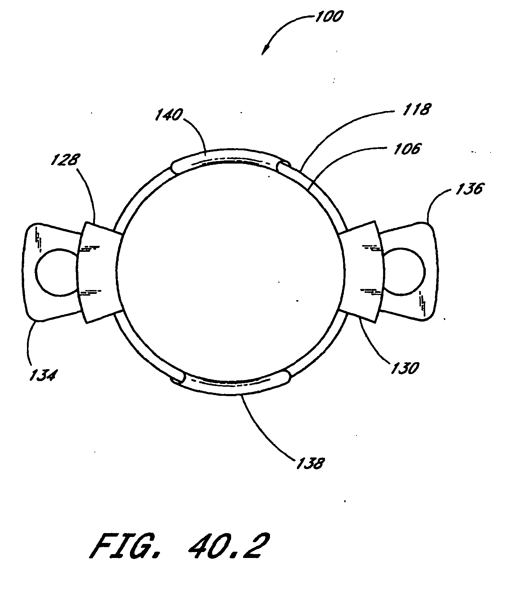

Figure 38.5 depicts a further embodiment of the lens system 100 in which the anterior translation members 110 and the posterior translation members 120 are paired in a number (in the example depicted, four) of separate positioners 1400 which are radially spaced, preferably equally radially spaced, about the optical axis. In the depicted embodiment, the anterior and posterior translation members 110,120 connect directly to the periphery of the viewing elements 106, 118; however, in other embodiments any of the connection techniques disclosed herein may be employed. As shown, the anterior translation members 100 preferably extend anteriorly from the periphery of the anterior viewing element before bending and extending posteriorly toward the apex/apices 112. As discussed above, this configuration is advantageous for promotion of fluid flow through an opening formed in the anterior aspect of the capsular bag 58. It has been found that the lens configuration shown in Figure 38.5 is well suited for the folding technique shown in Figures 40.1 and 40.2 below. In additional embodiments, the lens system 100 shown in Figure 38.5 may incorporate any other suitable features of the other embodiments of the lens system 100 disclosed herein, such as but not limited to the distending members and/or retention members detailed above.

### IX. IMPLANTATION METHODS

Various techniques may be employed in implanting the various embodiments of the lens system in the eye of a patient. The physician can first access the anterior aspect of the capsular bag 58 via any appropriate technique. Next, the physician incises the anterior of the bag; this may involve making the circular opening 66 shown in Figures 21 and 22, or the physician may make a "dumbbell" shaped incision by forming two small circular incisions or openings and connecting them with a third, straight-line incision. The natural lens is then removed from the capsular bag via any of various known techniques, such as phacoemulsification, cryogenic and/or radiative methods. To inhibit further cell growth, it is desirable to remove or kill all remaining epithelial cells. This can be achieved via cryogenic and/or radiative techniques, antimetabolites, chemical and osmotic agents. It is also possible to administer agents such as P15 to limit cell growth by sequestering the cells.

In the next step, the physician implants the lens system into the capsular bag. Where the lens system comprises separate anterior and posterior portions, the physician first folds or rolls the posterior portion and places it in the capsular bag through the anterior opening. After allowing the posterior portion to unroll/unfold, the physician adjusts the positioning of the posterior portion until it is within satisfactory limits. Next the physician rolls/folds and implants the anterior portion in a similar manner, and aligns and assembles the anterior portion to the posterior portion as needed, by causing engagement of mating portions, etc. formed on the anterior and posterior portions.

Where the lens system comprises anterior and posterior portions which are partially assembled or partially integral (see discussion above in the section titled MULTIPLE-PIECE AND OTHER EMBODIMENTS), the physician employs appropriate implantation procedures, subsequently folding/rolling and inserting those portions of the lens system that are separately foldable/rollable. In one embodiment, the physician first rolls/folds one portion of the partially assembled lens system and then inserts that portion. The physician then rolls/folds another portion of the partially assembled lens system and the inserts that portion. This is repeated until the entire system is inside the capsular bag, whereupon the physician completes the assembly of the portions and aligns the lens system as needed. In another embodiment, the physician first rolls/folds all of the separately rollable/foldable portions of the partially assembled lens system and then inserts the rolled/folded system into the capsular bag. Once the lens system is in the capsular bag, the physician completes the assembly of the portions and aligns the lens system as needed.

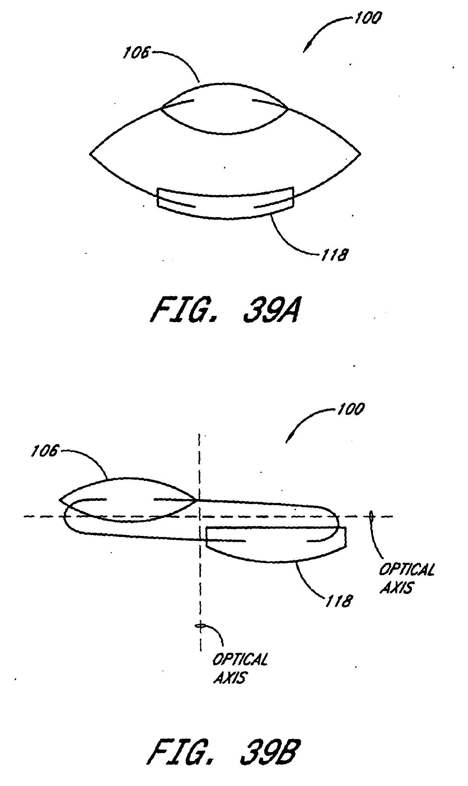

It is contemplated that conventional intraocular lens folding devices, injectors, syringes and/or shooters can be used to insert any of the lens systems disclosed herein. A preferred folding/rolling technique is depicted in Figure 39, where the lens system 100 is shown first in its normal condition (A). The anterior and posterior viewing elements 106, 118 are manipulated to place the lens system 100 in a low-profile condition (B), in which the viewing elements 106, 118 are out of axial alignment and are preferably situated so that no portion of the anterior viewing element 106 overlaps any portion of the posterior viewing element 118, as viewed along the optical axis. In the low-profile position (B), the thickness of the lens system 100 is minimized because the viewing elements 106, 118 are not "stacked" on top of each other, but instead have a side-by-side configuration. From the low-profile condition (B) the viewing elements 108, 118 and/or other portions of the lens system 100 can be folded or rolled generally about the transverse axis, or an axis parallel thereto. Alternatively, the lens system could be folded or rolled about the lateral axis or an axis parallel thereto. Upon folding/rolling, the lens system 100 is placed in a standard insertion tool as discussed above and is inserted into the eye.

When the lens system 100 is in the low-profile condition (B), the system may be temporarily held in that condition by the use of dissolvable sutures, or a simple clip which is detachable or manufactured from a dissolvable material. The sutures or clip hold the lens system in the low-profile condition during insertion and for a desired time after insertion. By temporarily holding the lens system in the low-profile condition after insertion, the sutures or clip provide time for fibrin formation on the edges of the lens system which, after the lens system departs from the low-profile condition, may advantageously bind the lens system to the inner surface of the capsular bag.

The physician next performs any adjustment steps which are facilitated by the particular lens system being implanted. Where the lens system is configured to receive the optic(s) in "open" frame members, the physician first observes/measures/determines the post-implantation shape taken on by the capsular bag and lens system in the accommodated and/or unaccommodated states and select(s) the optics which will provide the proper lens-system performance in light of the observed shape characteristics and/or available information on the patient's optical disorder. The physician then installs the optic(s) in the respective frame member(s); the installation takes place either in the capsular bag itself or upon temporary removal of the needed portion(s) of the lens system from the bag. If any portion is removed, a final installation and assembly is then performed with the optic(s) in place in the frame member(s).

Where the optic(s) is/are formed from an appropriate photosensitive silicone as discussed above, the physician illuminates the optic(s) (either anterior or posterior or both) with an energy source such as a laser until they attain the needed physical dimensions or refractive index. The physician may perform an intervening step of observing/measuring/determining the post-implantation shape taken on by the capsular bag and lens system in the accommodated and/or unaccommodated states, before determining any needed changes in the physical dimensions or refractive index of the optic(s) in question.

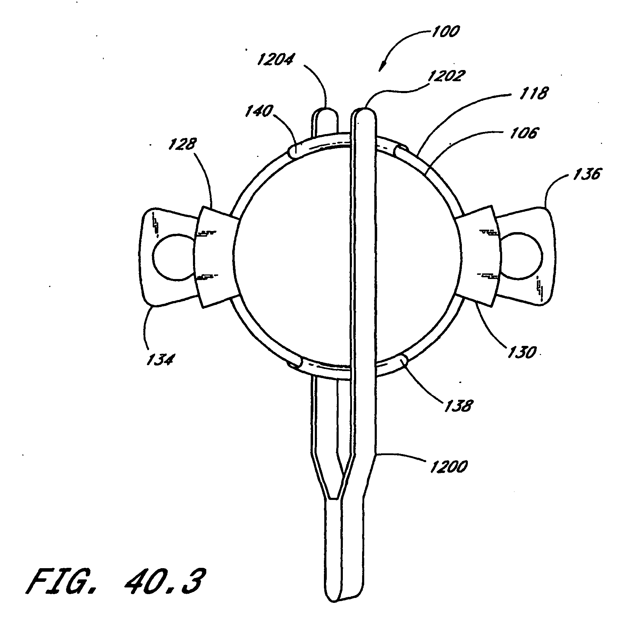

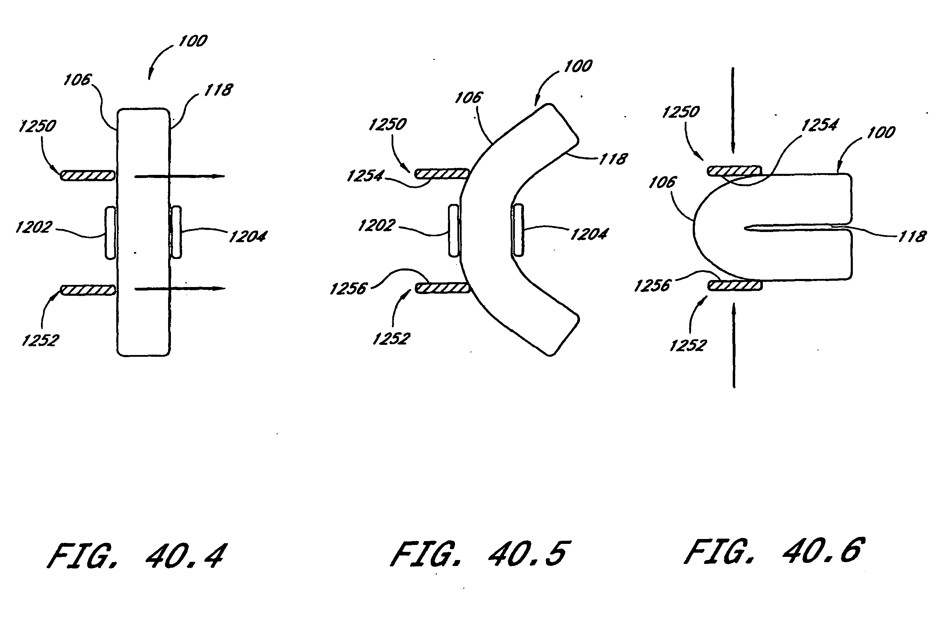

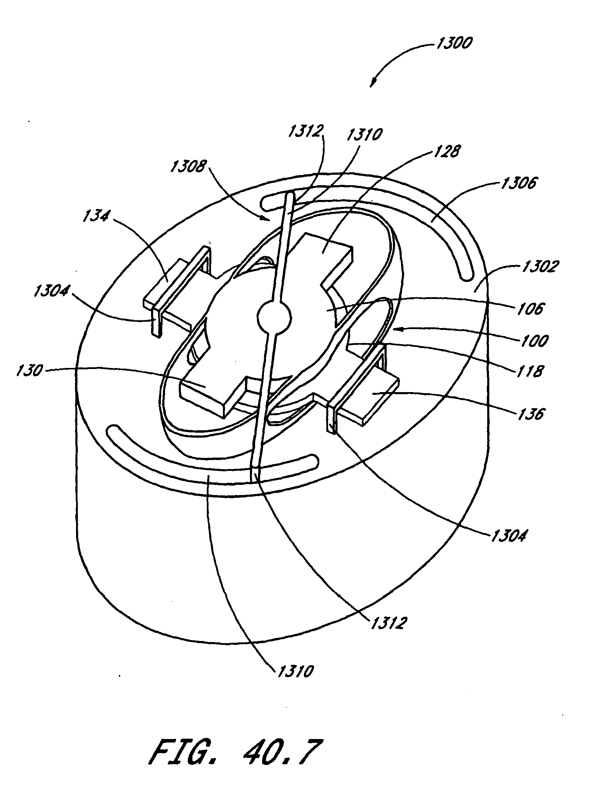

Figure 40 depicts a technique which may be employed during lens implantation to create a fluid flow path between the interior of the capsular bag 58 and the region of the eye anterior of the capsular bag 58. The physician forms a number of fluid-flow openings 68 in the anterior aspect of the capsular bag 58, at any desired location around the anterior opening 68. The fluid-flow openings 68 ensure that the desired flow path exists, even if a seal is created between the anterior opening 68 and a viewing element of the lens system.

Where an accommodating lens system is implanted, the openings 68 create a fluid flow path from the region between the viewing elements of the implanted lens system, and the region of the eye anterior of the capsular bag 58. However, the technique is equally useful for use with conventional (non-accommodating) intraocular lenses.

Figures 40.1 and 40.2 illustrate another embodiment of a method of folding the lens system 100. In this method the anterior viewing element 106 is rotated approximately 90 degrees about the optical axis with respect to the posterior viewing element 118. This rotation may be accomplished by applying rotational force to the upper edge of the first transition member 138 and the lower edge of the second transition member 140 (or vice versa), as indicated by the dots and arrows in Figure 40.1, while holding the posterior viewing element 118 stationary, preferably by gripping or clamping the distending members 134, 136. Alternatively, rotational force may be applied in a similar manner to a right edge of one of the retention members 128, 130 and to a left edge of the other of the retention members while holding the posterior viewing element 118 stationary. As still further alternatives, the anterior viewing element 106 could be held stationary while rotational force is applied to the posterior viewing element 118, at an upper edge of one of the distending members 134, 136 and at a lower edge of the other of the distending members; or both the anterior and posterior viewing elements 106, 118 could be rotated with respect to each other.

Preferably, the viewing elements 106, 118 are spread apart somewhat as the rotation is applied to the lens system so that the translation members and apices are drawn into the space between the viewing elements 106, 118 in response to the rotational force. Once the anterior viewing element 106 has been rotated approximately 90 degrees about the optical axis with respect to the posterior viewing element 118, the lens system 100 takes on the configuration shown in Figure 40.2, with the retention members 128, 130 generally radially aligned with the distending members 134, 136 and the translation members and apices disposed between the viewing elements 106, 118. This configuration is advantageous for inserting the lens system 100 into the capsular bag 58 because it reduces the insertion profile of the lens system 100 while storing a large amount of potential energy in the translation members. From the folded configuration the translation members thus exert a high "rebound" force when the lens system has been inserted to the capsular bag 58, causing the lens system to overcome any self-adhesion and spring back to the unfolded configuration shown in Figure 40.1 without need for additional manipulation by the physician.

Once the lens system 100 is in the folded configuration shown in Figure 40.2, it may be further folded and/or inserted into the capsular bag 58 by any suitable methods presently known in the art or hereafter developed. For example, as shown in Figure 40.3 the folding method may further comprise inserting the folded lens system 100 between the prongs 1202, 1204 of a clip 1200, preferably with the prongs 1202, 1204 oriented to extend along the transition members 138, 140, or along the retention members 128, 130 and the distending members 134, 136.

Figures 40.4 - 40.6 illustrate the use of jaws 1250,1252 of a pliers or forceps to fold the lens system 100 as it is held in the clip 1200. (Figures 40.4 - 40.6 show an end view of the clip-lens system assembly with the jaws 1250,1252 shown in section for clarity.) As shown in Figures 40.4 and 40.5, the edges of the jaws 1250, 1252 are urged against one of the anterior and posterior viewing elements 106, 118 while the jaws 1250, 1252 straddle the prong 1202 of the clip 1200. The resulting three-point load on the lens system 1200 causes it to fold in half as shown in Figure 40.5. As the lens system 100 approaches the folded configuration shown in Figure 40.6, the jaws 1250, 1252 slide into a pincer orientation with respect to the lens system 100, characterized by contact between the inner faces 1254, 1256 of the jaws 1250, 1252 and the anterior viewing element 106 or posterior viewing element 118. With such a pincer orientation established, the forceps may be used to grip and compress the lens system with inward-directed pressure and the clip 1200 can be withdrawn, as shown in Figure 40.6. With the lens system 100 thus folded, it can be inserted to the capsular bag 58 by any suitable method presently known in the art or hereafter developed.

Figure 40.7 depicts a folding tool 1300 which may be employed to fold the lens system 100 as discussed above in connection with figures 40.1 and 40.2. The tool 1300 includes a base 1302 with brackets 1304 which hold the lens system 100 to the base 1302 by gripping the distending members 134, 136. Formed within the base 1302 are arcuate guides 1306: The tool further comprises a rotor 1308 which in turn comprises a horizontal rod 1310 and integrally formed vertical rods 1312. The vertical rods 1312 engage the arcuate guides 1306, both of which have a geometric center on the optical axis of the lens system 100. The vertical rods 1312 and the arcuate guides 1306 thus coact to allow the horizontal rod to rotate at least 90 degrees about the optical axis of the lens system 100. The horizontal rod 1310 is fixed with respect to the anterior viewing element 106 of the lens system 100 so as to prevent substantially no relative angular movement between the rod 1310 and the anterior viewing element 106 as the rod 1310 (and, in turn, the anterior viewing element 106) rotates about the optical axis of the lens system 100. This fixed relationship may be established by adhesives and/or projections (not shown) which extend downward from the horizontal rod 1308 and bear against the upper edge of one of the transition members 138, 140 and against the lower edge of the other of the transition members as shown in Figure 40.1. As an alternative or as a supplement to this arrangement, the projections may bear against the retention members 128, 130 in a similar manner as discussed above.

Thus, when the rotor 1308 is advanced through its range of angular motion about the optical axis of the lens system 100, it forces the anterior viewing element 106 to rotate in concert therewith about the optical axis, folding the lens system as discussed above in connection with Figures 40.1 and 40.2. It is further contemplated that the folding tool 1300 may comprise the lower half of a package in which the lens system is stored and/or shipped to a customer, to minimize the labor involved in folding the lens system at the point of use. Preferably, the lens system is stored in the tool 1300 in the unfolded configuration, so as to avoid undesirable deformation of the lens system.

### X. THIN OPTIC CONFIGURATIONS

In some circumstances it is advantageous to make one or more of the optics of the lens system relatively thin, in order to facilitate rolling or folding, or to reduce the overall size or mass of the lens system. Discussed below are various optic configurations which facilitate a thinner profile for the optic; any one of these configurations may be employed as well as any suitable combination of two or more of the disclosed configurations.

One suitable technique is to employ a material having a relatively high index of refraction to construct one or more of the optics. In one embodiment, the optic material has an index of refraction higher than that of silicone. In another embodiment, the material has an index of refraction higher than about 1.43. In further embodiments, the optic material has an index of refraction of about 1.48, 1.49 or 1.55. In still further embodiments, the optic material has an index of refraction of about 1.43 to 1.55. By employing a material with a relatively high index of refraction, the curvature of the optic can be reduced (in other words, the radius/radii of curvature can be increased) thereby reducing the thickness of the optic without loss of focal power.

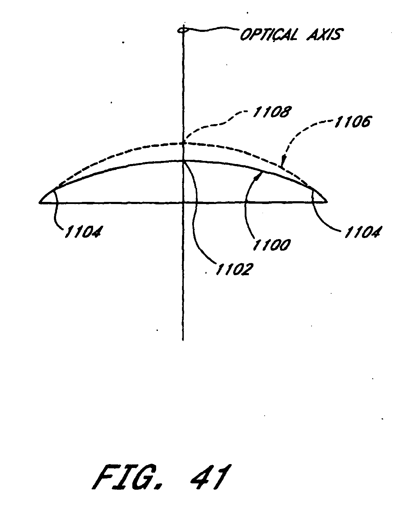

A thinner optic can also be facilitated by forming one or more of the surfaces of one or more of the optics as an aspheric surface, while maintaining the focal power of the optic. As shown in Figure 41, an aspheric, convex optic surface 1100 can be formed with the same radius of curvature (as a comparable-power spherical surface) at the vertex 1102 of the surface 1100 and a longer radius of curvature (with a common center point) at its periphery 1104, creating a thinner optic without sacrificing focal power. This contrasts with a spherical optic surface 1106, which is thicker at its vertex 1108 than is the aspheric surface 1102. In one embodiment, the thickness of the optic is reduced by about 19% at the vertex relative to a comparable-power spherical optic. It is contemplated that thinner, aspheric concave optic surfaces may be used as well. A further advantage of an aspheric optic surface is that it provides better image quality with fewer aberrations, and facilitates a thinner optic, than a comparable spherical surface.

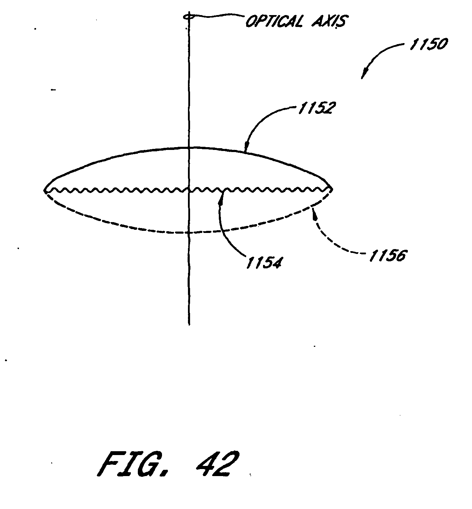

Figure 42 depicts a further strategy for providing a thinner optic 1150. The optic 1150 has a curved (spherical or aspheric) optic surface 1152 and a flat or planar (or otherwise less curved than a comparable refractive surface) diffractive optic surface 1154 in place of a second curved surface 1156. The diffractive optic surface 1154 can comprise any suitable diffraction grating, including the grooved surface depicted or any other diffractive surface presently known or hereafter developed, including holographic optical elements. By appropriately configuring the diffractive surface 1154 as is well known in the art, the optic 1150 can be made thinner than one having both curved surfaces 1152, 1154, while providing the same focal power. The use of the diffractive surface 1154 not only facilitates a thinner optic, but also reduces aberrations in the resulting image.

A further alternative for facilitating a thin, easy-to-fold optic is to employ, in place of a biconvex optic of refractive index greater than aqueous humor (i.e., greater than about 1.336), a biconcave optic of refractive index less than about 1.336, which is thinner at the optical axis than the biconvex optic. By constructing the biconcave optic of material having a refractive index less than about 1.336, the biconcave optic can be made to have the same effective focal power, when immersed in aqueous humor, as a given biconvex optic.

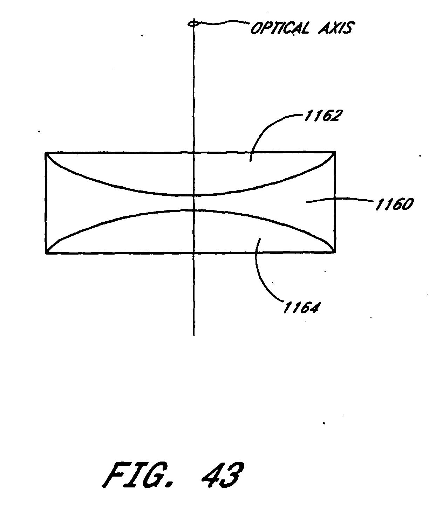

Still another alternative thin optic, shown in Figure 43, is a biconcave optic 1160 of low refractive index (for example, about 1.40 or less or about 1.336 or less) which is clad with first and second cladding portions 1162, 1164 constructed of higher-index material (for example, about 1.43 or greater). Such an optic can be made to have the same effective focal power, when immersed in aqueous humor, as a thicker biconvex optic.

As a further alternative, one or more of the surfaces of the optics may be formed as a multifocal surface, with spherical and/or aspheric focal regions. A multifocal surface can be made with less curvature than a comparable-power single-focus surface and thus allows the optic to be made thinner. The additional foci provide added power which replaces or exceeds the power that is "lost" when the surface is reduced in curvature. In one embodiment, the multifocal optic is constructed as a concentric-ring, refractive optic. In another embodiment, the multifocal optic is implemented as a diffractive multifocal optic.

Although this invention has been disclosed in the context of certain preferred embodiments and examples, it will be understood by those skilled in the art that the present invention extends beyond the specifically disclosed embodiments to other alternative embodiments and/or uses of the invention and obvious modifications and equivalents thereof. Thus, it is intended that the scope of the present invention herein disclosed should not be limited by the particular disclosed embodiments described above, but should be determined only by a fair reading of the claims that follow.

## Claims

1. An accommodating intraocular lens for implantation in an eye having an optical axis, said lens comprising:
an anterior portion comprised of a viewing element, said viewing element comprised of an optic having refractive power;
a posterior portion comprised of a viewing element, said viewing elements mounted to move relative to each other along the optical axis in response to force generated by the ciliary muscle of the eye; **characterized in that** it is provided with a retention portion comprising a first retention member attached to the anterior portion and having a free end sized and oriented to contact a portion of the lens capsule such that extrusion of the implanted lens through the lens capsule opening is inhibited.

2. The lens of Claim 1, wherein said retention portion is configured to displace the anterior aspect of the lens capsule anteriorly from said anterior viewing element and thereby prevent contact between said lens and an iris of said eye.

3. The lens of Claim 1, wherein said retention portion further comprises a second retention member having a fixed end attached to the anterior portion and a free end sized and oriented to contact a portion of the lens capsule, said fixed ends of said first and second retention members being attached to said viewing element of said anterior portion.

4. The lens of Claim 3, wherein said lens further comprises an optical axis which is adapted to be substantially coincident with the optical axis of the eye upon implantation of said lens, and said first and second retention portions are arranged 180 degrees apart from each other about said optical axis of said lens.

5. The lens of Claim 1, wherein said retention portion further comprises an opening formed therein to permit fluid flow therethrough.

6. The lens of Claim 1, wherein:
said lens further comprises first and second anterior translation members connected with respect to said viewing element of said anterior portion;
said viewing elements are mounted to move relative to each other along the optical axis between an accommodated state and an unaccommodated state;
said free end of said first retention member is anterior of at least a portion of at least one of said anterior translation members when said viewing elements are in said accommodated state; and
said viewing elements are spaced further apart when in the accommodated state than when in the unaccommodated state.

7. The lens of Claim 1, wherein:
said anterior viewing element has a periphery;
said anterior portion further comprises an anterior biasing element comprising first and second anterior translation members extending from said anterior viewing element;
said posterior viewing element has a periphery; and
said posterior portion further comprises a posterior biasing element comprising first and second posterior translation members extending from said posterior viewing element;
said first anterior translation member and said first posterior translation member meeting at a first apex of said intraocular lens, and said second anterior translation member and said second posterior translation member meeting at a second apex of the intraocular lens;
wherein each of said translation members is attached to one of said viewing elements at at least one attachment location, all of the attachment locations being significantly further away from the apices than the peripheries of the viewing elements are from the apices.

8. The lens capsule of Claim I wherein said anterior portion is adapted to contact portions of the lens capsule while being spaced from the lens capsule in at least one location so as to provide a fluid flow channel that extends from a region between said viewing elements to a region outside said capsule.

9. The lens of Claim 8, wherein said anterior portion comprises an anterior biasing element connected to a periphery of said anterior viewing element so that said periphery of said anterior viewing element is spaced from an inner surface of the lens capsule upon implantation of said lens, and said fluid flow channel is defined by said periphery, said anterior biasing element, said inner surface of said lens capsule and said capsule opening.

10. The lens of Claim 9, further comprising a first retention member having a fixed end connected to said periphery of said anterior viewing element and a free end spaced from said fixed end, said fluid flow channel being defined by said periphery of said anterior viewing element, said anterior biasing element, said first retention member, said inner surface of said lens capsule and said capsule opening.

11. The lens of Claim 8, wherein:
said anterior portion comprises an anterior biasing element connected to a periphery of said anterior viewing element via first and second transition members extending from said periphery;
said lens further comprises first and second retention members each having a fixed end connected to said periphery of said anterior viewing element and a free end spaced from said fixed end;
each of said first and second transition members is located between and angularly spaced from said first and second retention members; and
at least one of said transition members and said anterior biasing element is adapted to contact an inner surface of said lens capsule near said capsule opening and thereby maintain said periphery of said anterior viewing element in spaced relation to said inner surface of said lens capsule;
said fluid flow channel being defined by said periphery, said transition members, said retention members, said inner surface and said anterior opening.

12. The lens of Claim 8, wherein:
said anterior viewing element has a periphery;
said anterior portion further comprises an anterior biasing element comprising first and second anterior translation members extending from said anterior viewing element;
said posterior viewing element has a periphery; and
said posterior portion further comprises a posterior biasing element comprising first and second posterior translation members extending from said posterior viewing element;
said first anterior translation member and said first posterior translation member meeting at a first apex of said intraocular lens, and said second anterior translation member and said second posterior translation member meeting at a second apex of the intraocular lens;
wherein each of said translation members is attached to one of said viewing elements at at least one attachment location, all of the attachment locations being significantly further away from the apices than the peripheries of the viewing elements are from the apices.

13. The lens of Claim 1, wherein:
said lens system further comprises an optical axis which is adapted to be substantially coincident with the optical axis of the eye upon implantation of said lens system;
said anterior portion and said posterior portion meet at first and second apices of said lens system; and
said first retention member has a radically outermost portion which is significantly closer to said optical axis of said lens system than is either of said first and second apices.

14. The lens of Claim 1, wherein:
said viewing elements are interconnected by plural biasing elements, said biasing elements forming first and second apices of said lens system, one of said biasing elements being connected to said anterior viewing element at at least first and second biasing element connection locations; and
the first retention member is connected to a periphery of said anterior viewing element at a retention member connection location and extending anteriorly from said periphery, said retention member connection location being separate from said at least first and second biasing element connection locations.

15. The lens of Claim 1, wherein in use the retention portion displaces the anterior aspect of the lens capsule anteriorly from said anterior viewing element and thereby prevents contact between the lens and an iris of the eye.

16. The lens of Claim 1, wherein in use the retention portion contacts a portion of the lens capsule to space the anterior portion from the lens capsule in at least one location so as to provide a fluid flow channel that extends from a region between said viewing elements to a region outside said capsule.

17. The lens of Claim 1, wherein the intraocular lens consists of a single, integral unit.

18. The lens of Claim 1, wherein at least a portion of the first retention member extends anterior to the anterior viewing element.

## Patentansprüche

1. Akkomodierende Intraokularlinse zur Implantation in ein Auge mit einer optischen Achse, wobei die Linse aufweist:
einen anterioren Teil, der ein Betrachtungselement aufweist, wobei das Betrachtungselement eine Optik mit einer Brechkraft aufweist,
einen posterioren Teil, der aus einem Betrachtungselement besteht, wobei die Betrachtungselemente so montiert sind, daß sie sich in Reaktion auf eine Kraft, die durch den Ziliarmuskel des Auges erzeugt wird, relativ zueinander entlang der optischen Achse bewegen, **dadurch gekennzeichnet, daß** sie mit einem Rückhalteteil versehen ist, welcher ein erstes Rückhalteelement aufweist, welches an dem anterioren Teil angebracht ist und ein freies Ende aufweist, das so bemessen und ausgerichtet Ist, daß es mit einem Teil der Linsenkapsel so in Kontakt tritt, daß ein Durchdrücken der implantierten Linse durch die Linsenkapselöffnung verhindert wird.

2. Linse nach Anspruch 1, wobei der Rückhalteteil so ausgestaltet ist, daß er die anteriore Seite der Linsenkapsel von dem anterioren Betrachtungselement anterior verlagert und dadurch einen Kontakt zwischen der Linse und einer Iris des Auges verhindert.

3. Linse nach Anspruch 1, wobei der Rückhalteteil weiterhin ein zweites Rückhalteelement aufweist, welches ein fixiertes Ende hat, das an dem anterioren Teil angebracht ist, und ein freies Ende hat, das so bemessen und ausgerichtet ist, daß es mit einem Teil der Linsenkapsel in Kontakt kommt, wobei die fixierten Enden der ersten und zweiten Rückhalteelemente an dem Betrachtungselement des anterioren Teils angebracht sind.

4. Linse nach Anspruch 3, wobei die Linse weiterhin eine optische Achse aufweist, die so ausgestaltet ist, daß sie bei Implantation der Linse im wesentlichen auf der optischen Achse des Auges liegt, und wobei die ersten und zweiten Rückhalteteile um die optische Achse der Linse um etwa 180 Grad voneinander entfernt angeordnet sind.

5. Linse nach Anspruch 1, wobei der Rückhalteteil weiterhin eine darin ausgebildete Öffnung aufweist, um einen Fluidfluß durch diesen hindurch zu gestatten.

6. Linse nach Anspruch 1, wobei:
die Linse weiterhin erste und zweite anteriore Translationselemente aufweist, die in Bezug auf das Betrachtungselement des anterioren Teils verbunden sind,
die Betrachtungselemente so montiert sind, daß sie sich entlang der optischen Achse zwischen einem akkommodierten Zustand und einem nicht-akkommodierben Zustand relativ zueinander bewegen,
das freie Ende des ersten Rückhalteelements vor wenigstens einem Tell wenigstens eines der anterioren Translationselemente liegt wenn die Betrachtungselemente in dem akkommodierten Zustand sind, und
die Betrachtungselemente Im akkommodierten Zustand weiter voneinander beabstandet sind als im nicht-akkommodierten Zustand.

7. Linse nach Anspruch 1, wobei:
das anteriore Betrachtungselement eine Peripherie hat,
der anteriore Teil weiterhin ein anteriores Vorspannelement aufweist, welches erste und zweite anteriore Translationselemente beinhaltet die sich von dem anterioren Betrachtungselement erstrecken,
das posteriore Betrachtungselement eine Peripherie hat und
der posteriore Teil weiterhin ein posteriores Vorspannelement aufweist, welches erste und zweite posteriore Translationselemente beinhaltet die sich von dem posterioren Betrachtungselement aus erstrecken,
das erste anteriore Translatlonselement und das erste posteriore Translationselement an einem ersten Apex der Intraokularlinse aufeinandertreffen und das zweite anteriore Translationselement und das zweite posteriore Translationselement an einem zweiten Apex der Intraokularlinse aufeinandertreffen,
wobei jedes der Translationselemente an wenigstens einer Befestigungsstelle an einem der Betrachtungselemente angebracht ist, wobei alle Befestigungsstellen in signifikantem Maße weiter von den Apices entfernt liegen als die Peripherien der Betrachtungselemente von den Apices entfernt liegen.

8. Linsenkapsel nach Anspruch 1, wobei der anteriore Teil so ausgestaltet ist, daß er mit Teilen der Linsenkapsel in Kontakt tritt, während er an wenigstens einer Stelle von der Linsenkapsel beabstandet ist, um einen Fluidflußkanal bereitzustellen, welcher sich von einem Bereich zwischen den Betrachtungselementen zu einem Bereich außerhalb der Kapsel erstreckt.

9. Linse nach Anspruch 8, wobei der anteriore Teil ein anteriores Vorspannelement aufweist, welches mit einer Peripherie des anterioren Betrachtungselements so verbunden ist, daß die Peripherie des anterioren Betrachtungselements bei Implantation der Linse von einer Inneren Oberfläche der Linsenkapsel beabstandet ist, und der Fluidflußkanal durch die Peripherie, das anteriore Vorspannelement, die innere Oberfläche der Linsenkapsel und die Kapselöffnung gebildet wird.

10. Linse nach Anspruch 9, welche weiterhin ein erstes Rückhalteelement aufweist, welches ein fixiertes Ende hat, das mit der Peripherie des anterioren Betrachtungselements verbunden ist, und ein freies Ende hat, das von dem fixierten Ende beabstandet ist, wobei der Fluidflußkanal durch die Peripherie des anterioren Betrachtungselements, das anteriore Vorspannelement, das erste Rückhalteelement, die innere Oberfläche der Linsenkapsel und die Kapselöffnung gebildet wird.

11. Linse nach Anspruch 8, wobei:
der anteriore Teil ein anteriores Vorspannelement aufweist, welches mit einer Peripherie des anterioren Betrachtungselements über erste und zweite Übergangselemente, die sich von der Peripherie erstrecken, verbunden ist,
die Linse weiterhin erste und zweite Rückhalteelemente aufweist die jeweils ein fixiertes Ende haben, das mit der Peripherie des anterioren Betrachtungselements verbunden ist, und ein freies Ende haben, das von dem fixierten Ende beabstandet ist,
jedes der ersten und zweiten Übergangselemente zwischen den ersten und zweiten Rückhalteelementen liegt und um einen Winkel von diesen beabstandet ist, und
wenigstens eines der Übergangselemente und das anteriore Vorspannelement so ausgestaltet sind, daß sie mit einer inneren Oberfläche der Linsenkapsel in der Nähe der Kapselöffnung in Kontakt kommen und **dadurch** die Peripherie des anterioren Betrachtungselements In einer Abstandsbeziehung zu der Inneren Oberfläche der Linsenkapsel halten,
wobei der Fluidflußkanal durch die Peripherie, die Übergangselemente, die Rückhalteelemente, die innere Oberfläche und die anteriore Öffnung gebildet wird.

12. Linse nach Anspruch 8, wobei:
das anteriore Betrachtungselement eine Peripherie hat,
der anteriore Teil ein anteriores Vorspannelement aufweist, welches erste und zweite anteriore Translationselemente beinhaltet, die sich von dem anterioren Betrachtungselement erstrecken,
das posteriore Betrachtungselement eine Peripherie hat und
der posteriore Teil weiterhin ein posteriores Vorspannelement aufweist, welches erste und zweite posteriore Translationselemente beinhaltet, die sich von dem posterioren Betrachtungselement erstrecken,
das erste anteriore Translationselement und das erste posteriore Translationselement an einem ersten Apex der intraokularlinse aufeinandertreffen und das zweite anteriore Translationselement und das zweite posteriore Translationselement an einem zweiten Apex der Intraokularlinse aufeinandertreffen,
wobei jedes der Translationselemente an wenigstens einer Befestigungsstelle an einem der Betrachtungselemente angebracht ist, wobei alle Befestigungsstellen in signifikantem Maße weiter von den Apices entfernt liegen als die Peripherien der Betrachtungselemente von den Apices entfernt liegen.

13. Linse nach Anspruch 1, wobei:
das Linsensystem weiterhin eine optische Achse aufweist, die so ausgestaltet ist, daß sie bei implantation des Linsensystems im wesentlichen auf der optischen Achse des Auges liegt,
der anteriore Teil und der posteriore Teil an ersten und zweiten Apices des Linsensystems aufeinandertreffen und
das erste Rückhalteelement einen radial äußersten Abschnitt hat, welcher in signifikantem Maße näher bei der optischen Achse des Linsensystems liegt als je einer der ersten und zweiten Apices.

14. Linse nach Anspruch 1, wobei:
die Betrachtungselemente durch mehrere Vorspannelemente miteinander verbunden sind, wobei die Vorspannelemente erste und zweite Apices des Linsensystems bilden, wobei eines der Vorspannelemente an wenigstens ersten und zweiten Vorspannelement-Verbindungsstellen mit dem anterioren Betrachtungselement verbunden ist, und
das erste Rückhalteelement an einer Rückhalteelement-Verbindungsstelle mit einer Peripherie des anterioren Betrachtungselements verbunden ist und sich von der Peripherie anterior erstreckt, wobei die Rückhalteelement-Verbindungsstelle von den wenigstens ersten und zweiten Vorspannelement-Verbindungsstellen getrennt ist.

15. Linse nach Anspruch 1, wobei im Gebrauch der Rückhaltetell die anteriore Seite der Linsenkapsel von dem anterioren Betrachtungselement anterior verlagert und **dadurch** einen Kontakt zwischen der Linse und einer Iris des Auges verhindert

16. Linse nach Anspruch 1, wobei im Gebrauch der Rückhalteteil mit einem Teil der Linsenkapsel so in Kontakt tritt, daß der anteriore Teil von der Linsenkapsel an wenigstens einer Stelle beabstandet ist, um einen Fluidflußkanal bereitzustellen, der sich von einer Region zwischen den Betrachtungselementen zu einer Region außerhalb der Kapsel erstreckt.

17. Linse nach Anspruch 1, wobei die Intraokularlinse aus einer einzigen Integralen Einheit besteht

18. Linse nach Anspruch 1, wobei sich wenigstens ein Teil des ersten Rückhalteelements anterior zu dem anterioren Betrachtungselement erstreckt.

## Revendications

1. Lentille intraoculaire d'accommodation destinée à être implantée dans un oeil ayant un axe optique, ladite lentille comportant :
une partie antérieure constituée d'un élément de vision, ledit élément de vision étant constitué d'une optique ayant une puissance de réfraction ;
une partie postérieure constituée d'un élément de vision, lesdits éléments de vision étant montés de façon à se déplacer l'un par rapport à l'autre le long de l'axe optique en réponse à une force générée par le muscle ciliaire de l'oeil ; **caractérisée en ce qu'**elle est pourvue d'une partie de retenue comprenant un premier élément de retenue attaché à la partie antérieure et ayant une extrémité libre dimensionnée et orientée pour entrer en contact avec une partie de la capsule du cristallin afin d'empêcher une extrusion de la lentille implantée à travers l'ouverture de la capsule du cristallin.

2. Lentille selon la revendication 1, dans laquelle ladite partie de retenue est configurée pour déplacer la face antérieure de la capsule du cristallin du côté antérieur depuis ledit élément de vision antérieur et empêcher ainsi un contact entre ladite lentille et l'iris dudit oeil.

3. Lentille selon la revendication 1, dans laquelle ladite partie de retenue comporte en outre un second élément de retenue ayant une extrémité fixe qui est attachée à la partie antérieure et une extrémité libre dimensionnée et orientée de manière à entrer en contact avec une partie de la capsule du cristallin, lesdites extrémités fixes desdits premier et second éléments de retenue étant attachées audit élément de vision de ladite partie antérieure.

4. Lentille selon la revendication 3, laquelle lentille comporte en outre un axe optique qui est adapté de façon à être sensiblement coïncidant avec l'axe optique de l'oeil lors d'une implantation de ladite lentille, et lesdites première et seconde parties de retenue sont agencées à 180 degrés l'une par rapport à l'autre autour dudit axe optique de ladite lentille.

5. Lentille selon la revendication 1, dans laquelle ladite partie de retenue comporte en outre une ouverture formée en elle pour permettre un écoulement de fluide à travers elle.

6. Lentille selon la revendication 1, dans laquelle :
ladite lentille comporte en outre des premier et second éléments antérieurs de translation reliés par rapport audit élément de vision de ladite partie antérieure ;
lesdits éléments de vision sont montés de façon à se déplacer l'un par rapport à l'autre le long de l'axe optique entre un état d'accommodation et un état de non accommodation ;
ladite extrémité libre dudit premier élément de retenue est antérieure par rapport à au moins une partie d'au moins l'un desdits éléments antérieurs de translation lorsque lesdits éléments de vision sont dans ledit état d'accommodation ; et
lesdits éléments de vision sont plus espacés l'un de l'autre lorsqu'ils sont dans l'état d'accommodation que lorsqu'ils sont dans l'état de non accommodation.

7. Lentille selon la revendication 1, dans laquelle :
ledit élément antérieur de vision a une périphérie ;
ladite partie antérieure comporte en outre un élément antérieur de sollicitation comprenant des premier et second éléments antérieurs de translation s'étendant depuis ledit élément antérieur de vision ;
ledit élément postérieur de vision a une périphérie ; et
ladite partie postérieure comporte en outre un élément postérieur de sollicitation comprenant des premier et second éléments postérieurs de translation s'étendant depuis ledit élément postérieur de vision ;
ledit premier élément antérieur de translation et ledit premier élément postérieur de translation se rejoignant à un premier sommet de ladite lentille intraoculaire, et ledit second élément antérieur de translation et ledit second élément postérieur de translation se rejoignant à un second sommet de la lentille intraoculaire ;
dans laquelle chacun desdits éléments de translation est attaché à l'un desdits éléments de vision en au moins un emplacement d'attache, tous les emplacements d'attache étant notablement plus éloignés des sommets que les périphéries des éléments de vision le sont des sommets.

8. Lentille selon la revendication 1, dans laquelle ladite partie antérieure est conçue pour entrer en contact avec des parties de la capsule du cristallin tout en étant espacée de la capsule du cristallin en au moins un emplacement afin de former un canal d'écoulement de fluide qui s'étend depuis une région située entre lesdits éléments de vision jusqu'à une région à l'extérieur de ladite capsule.

9. Lentille selon la revendication 8, dans laquelle ladite partie antérieure comporte un élément antérieur de sollicitation relié à une périphérie dudit élément antérieur de vision afin que ladite périphérie dudit élément antérieur de vision soit espacée d'une surface intérieure de la capsule du cristallin lors d'une implantation de ladite lentille et ledit canal d'écoulement de fluide est défini par ladite périphérie, ledit élément antérieur de sollicitation, ladite surface intérieure de ladite capsule du cristallin et ladite ouverture de la capsule.

10. Lentille selon la revendication 9, comportant en outre un premier élément de retenue ayant une extrémité fixe reliée à ladite périphérie dudit élément antérieur de vision et une extrémité libre espacée de ladite extrémité fixe, ledit canal d'écoulement de fluide étant défini par ladite périphérie dudit élément antérieur de vision, par ledit élément antérieur de sollicitation, par ledit premier élément de retenue, par ladite surface intérieure de ladite capsule du cristallin et par ladite ouverture de la capsule.

11. Lentille selon la revendication 8, dans laquelle :
ladite partie antérieure comporte un élément antérieur de sollicitation relié à une périphérie dudit élément antérieur de vision par l'intermédiaire de premier et second éléments de transition s'étendant depuis ladite périphérie ;
ladite lentille comportant en outre des premier et second éléments de retenue ayant chacun une extrémité fixe reliée à ladite périphérie dudit élément antérieur de vision et une extrémité libre espacée de ladite extrémité fixe ;
chacun desdits premier et second éléments de transition est situé entre lesdits premier et second éléments de retenue et en est espacé angulairement ; et
au moins l'un des éléments de transition et dudit élément antérieur de sollicitation est conçu pour entrer en contact avec une surface intérieure de ladite capsule du cristallin à proximité de ladite ouverture de la capsule et pour maintenir ainsi ladite périphérie dudit élément antérieur de vision à distance de ladite surface intérieure de ladite capsule du cristallin ;
ledit canal d'écoulement de fluide étant défini par ladite périphérie, lesdits éléments de transition, les éléments de retenue, ladite surface intérieure et ladite ouverture antérieure.

12. Lentille selon la revendication 8, dans laquelle :
ledit élément antérieur de vision a une périphérie ;
ladite partie antérieure comporte en outre un élément antérieur de sollicitation comprenant des premier et second éléments antérieurs de translation s'étendant depuis ledit élément antérieur de vision ;
ledit élément postérieur de vision a une périphérie ; et
ladite partie postérieure comporte en outre un élément postérieur de sollicitation comprenant des premier et second éléments postérieurs de translation s'étendant depuis ledit élément postérieur de vision ;
ledit premier élément antérieur de translation et ledit premier élément postérieur de translation se rejoignant à un premier sommet de ladite lentille intraoculaire, et ledit second élément antérieur de translation et ledit second élément postérieur de translation se rejoignant à un second sommet de la lentille intraoculaire ;
dans laquelle chacun desdits éléments de translation est attaché à l'un desdits éléments de vision en au moins un emplacement d'attache, tous les emplacements d'attache étant notablement plus éloignés des sommets que les périphéries des éléments de vision le sont des sommets.

13. Lentille selon la revendication 1, dans laquelle :
ledit système de lentille comporte en outre un axe optique qui est conçu pour coïncider sensiblement avec l'axe optique de l'oeil lors de l'implantation dudit système de lentille ;
ladite partie antérieure et ladite partie postérieure se rejoignent en des premier et second sommets dudit système de lentille ; et
ledit premier élément de retenue a une partie située radicalement le plus à l'extérieur qui est notablement plus proche dudit axe optique dudit système de lentille que ne n'est chacun desdits premier et second sommets.

14. Lentille selon la revendication 1, dans laquelle :
lesdits éléments de vision sont reliés entre eux par plusieurs éléments de sollicitation, lesdits éléments de sollicitation formant des premier et second sommets dudit système de lentille, l'un desdits éléments de sollicitation étant relié audit élément antérieur de vision en au moins des premier et second emplacements de liaison de l'élément de sollicitation ; et
le premier élément de retenue est relié à une périphérie dudit élément antérieur de vision en un emplacement de liaison d'élément de retenue et s'étendant antérieurement depuis ladite périphérie, ledit emplacement de liaison de l'élément de retenue étant séparé desdits au moins premier et second emplacements de liaison de l'élément de sollicitation.

15. Lentille selon la revendication 1, dans laquelle, lors de l'utilisation, la partie de retenue déplace la face antérieure de la capsule du cristallin du côté antérieur à partir dudit élément antérieur de vision et empêche ainsi tout contact entre la lentille et l'iris de l'oeil.

16. Lentille selon la revendication 1, dans laquelle, lors de l'utilisation, la partie de retenue entre en contact avec une partie de la capsule du cristallin pour espacer la partie antérieure de la capsule du cristallin en au moins un emplacement afin de former un canal d'écoulement de fluide qui s'étend depuis une région située entre lesdits éléments de vision jusqu'à une région à l'extérieur de ladite capsule.

17. Lentille selon la revendication 1, laquelle lentille intraoculaire est constituée d'une seule pièce intégrée.

18. Lentille selon la revendication 1, dans laquelle au moins une partie du premier élément de retenue s'étend du côté antérieur par rapport à l'élément antérieur de vision.
